# FASCICULE DE BREVET EUROPEEN

(11) **EP 2 173 189 B1**
(45) Date de publication et mention de la délivrance du brevet: **06.03.2019**
(21) Numéro de dépôt: 08836060.7
(22) Date de dépôt: 18.07.2008
(51) Int. Cl.: A61K 9/16, A61K 9/50, A61K 47/36, A61K 47/38, A23K 40/10, A23K 40/30, A23K 10/18, A23K 20/179, A23K 20/163, A23K 20/189, A23K 20/195

(54) **COMPOSITIONS PULVERULENTES STABLES ET LEUR PROCÉDÉ DE PRÉPARATION**
STABILE PULVERZUSAMMENSETZUNGEN UND VERFAHREN ZU DEREN HERSTELLUNG
STABLE POWDER COMPOSITIONS AND THEIR METHOD OF PREPARATION

(30) Priorité: 20.07.2007 FR 0705307
(43) Date de publication de la demande: 14.04.2010
(73) Titulaire: Innov'ia, 17000 La Rochelle (FR)
(72) Inventeur: BUISSON, Pierre, F-17140 Lagord (FR)
(74) Mandataire: Grosset-Fournier, Chantal Catherine
(86) Numéro de dépôt international: PCT/FR2008/001069
(87) Numéro de publication internationale: WO 2009/043988

(56) Documents cités:
- EP-A- 0 600 775
- WO-A-03/059086
- WO-A-2005/074707
- KIM H S ET AL: "METHOD FOR THE PREPARATION OF STABILE MICROENCAPSULATED LACTIC ACID BACTERIA" JOURNAL OF INDUSTRIAL MICROBIOLOGY, SOCIETY FOR INDUSTRILA MICROBIOLOGY, vol. 3, no. 4, 1 janvier 1988 (1988-01-01), pages 253-257, XP009084110 ISSN: 0169-4146
- XIAO HONG YANG ET AL: "Viscosity properties of sodium carboxymethylcellulose solutions", CELLULOSE, KLUWER ACADEMIC PUBLISHERS (DORDRECHT), NL, vol. 14, no. 5, 29 June 2007 (2007-06-29), pages 409-417, XP019524883, ISSN: 1572-882X, DOI: 10.1007/S10570-007-9110-7

## Description

L'invention concerne un nouveau procédé de préparation de compositions pulvérulentes stables. En particulier, l'invention concerne un nouveau procédé de préparation de compositions pulvérulentes comportant une substance sensible, notamment à la température et/ou à la pression et/ou à l'humidité relative.

Les substances sensibles sont fréquemment des substances actives utilisées en pharmacologie ou dans des compositions utilisées dans différents domaines tels que l'alimentation humaine ou animale, les détergents, etc. A titre d'exemple, on peut citer les protéines, notamment les enzymes, les vitamines, les bactéries, les levures, les anti-oxydants, les caroténoïdes, les huiles essentielles ou encore des substances pharmacologiquement actives telles que les antibiotiques.

Cependant, lors de la préparation de ces compositions et notamment lors des opérations de granulation, les substances sont soumises à de nombreuses contraintes. En effet, la caractéristique principale des machines réalisant ces opérations est d'obliger les matières constituant la composition finale à passer à travers une filière présentant des perforations de différentes tailles, créant ainsi un cisaillement important conduisant à une montée en température de la matière à coeur. De plus, pour faciliter ce passage, il est classiquement effectué un préchauffage de la matière et de la vapeur d'eau est également ajoutée pour apporter une cohésion. Ces différentes contraintes de température, de pression et d'humidité peuvent être nuisibles pour des substances sensibles, qui peuvent alors perdre une partie de leur activité lors de ces opérations.

Pour résoudre ces problèmes, différentes solutions ont pu être proposées par le passé.

Ainsi, le brevet EP 0 569 468 propose une méthode de préparation de granulés contenant une(des) enzyme(s) destinés à l'alimentation animale et qui sont enrobées avec une graisse ou une cire présentant un point de fusion élevé, afin d'augmenter la résistance aux conditions de granulation. Cependant, un tel enrobage présente le désavantage d'entraîner un temps de dissolution long des granulés, ce qui diminue la biodisponibilité des enzymes pour l'animal.

De même, la demande WO 00/47060 décrit un procédé de préparation de granulés contenant une(des) enzyme(s) enrobées avec du polyéthylène glycol. Cet enrobage permet alors d'augmenter la stabilité des enzymes lors d'une étape de granulation mais uniquement en présence d'additifs tels que des sels inorganiques hydrosolubles (par exemple ZnSO₄) ou encore le tréhalose. Ces additifs ne représentent pas nécessairement un intérêt pour l'utilisation envisagée de ces granulés et ne fait qu'en augmenter le coût.

Une méthode de préparation de granulés contenant une(des) enzyme(s) enrobés avec une polyoléfine a également été développée dans la demande WO 03/059087, notamment pour une utilisation dans le domaine de l'alimentation animale. L'enrobage utilisé permet alors d'augmenter la stabilité de la(des) enzyme(s) lors des étapes de granulation notamment. Cependant, cela nécessite l'utilisation de dérivés polymériques qui ne sont pas nécessaires à l'alimentation de l'animal et qui augmentent le coût de fabrication des granulés.

La demande WO2005/074707 décrit des formulations stabilisées de phosphatase, l'agent de stabilisation étant choisi exclusivement parmi l'agar-agar, l'alginate, la carraghénine, le furcelleran, la gomme ghatti, la gomme adragante, la gomme karya, la gomme de guarana, la gomme de caroube, la gomme de tamarin, l'arabinogalactane et la gomme xanthane. Ces formulations peuvent être enrobées par des polymères notamment choisis parmi les dérivés cellulosiques ayant un poids moléculaire compris entre 6000 et 80000. Cette demande reste silencieuse sur la viscosité des produits utilisés.

La demande EP0600775 décrit des principes actifs stabilisés par enrobage. La composition d'enrobage comprenant un agent filmogène qui peut notamment être un dérivé cellulosique comme l'acétate de cellulose, l'éthylcellulose ou la méthylcellulose et un agent porogène qui permet une destruction rapide de ladite composition d'enrobage en constituant des points d'accès préférentiels aux fluides biologiques.

La demande WO03/059086 décrit une formulation de granule et son mode de préparation. Toutefois aucune information n'est donnée sur la viscosité des produits utilisés.

Un des aspects de l'invention est donc de proposer un nouveau procédé de préparation de compositions pulvérulentes contenant au moins une substance sensible qui puisse résister à des conditions de températures, de pressions et d'humidité relative élevées et qui soit stable pendant le transport et le stockage, ce procédé étant facile à mettre en oeuvre et peu coûteux.
La présente invention a plus spécialement pour objet l'utilisation d'au moins un composé visqueux, dont la viscosité est supérieure à 5 Pa.s, pour empêcher substantiellement la dégradation d'une substance sensible contenue dans une composition pulvérulente stable, ladite substance sensible subissant moins de 20% de dégradation lorsque ladite composition pulvérulente stable est soumise à des températures de 60 °C à 100 °C et/ou à des pressions de 2.10⁶ à 10⁷ Pa et/ou à une humidité relative de 60% à 100% pendant l'étape de granulation de ladite composition, ladite composition pulvérulente contenant des particules comprenant :
- un coeur contenant ladite substance sensible et au moins un composé visqueux en tant qu'agent d'imprégnation de la substance sensible, et au moins un support de la substance sensible, et
- un enrobage du coeur, lequel enrobage contient au moins un composé visqueux en tant qu'agent d'engluage,
lesdits composés visqueux étant choisis parmi la carboxyméthylcellulose (CMC).
Les composés visqueux utilisés dans la présente invention présentent l'intérêt d'être quasiment hydrophobes lorsqu'ils sont soumis à des pressions élevées, qui sont de l'ordre de 10⁶ à 10⁷ Pa, mais retrouvent toutes leurs propriétés de solubilité lorsqu'ils sont remis en solution. Cela permet ainsi de protéger les substances sensibles lors de la préparation de granulés par une étape de granulation tout en garantissant par la suite une bonne biodisponibilité des substances sensibles grâce à des temps courts de dissolution des particules de la composition pulvérulente. Notamment, lorsque les compositions pulvérulentes sont destinées à l'alimentation animale, la substance sensible, qui est aussi le principe actif, sera libérée directement dans le tractus digestif de l'animal.
Par « granulation », on entend une étape permettant la préparation de granulés se présentant sous forme de cylindres de 1 à 10 mm de diamètre et de 1 à 5 cm de longueur par passage dans une filière. Cette étape est caractérisée par des contraintes de température et pression élevées et de cisaillement, ainsi que par la présence de vapeur d'eau, ces contraintes pouvant détériorer des substances sensibles.
Les mesures de viscosité sont réalisées à l'aide d'un viscosimètre BROOKFIELD modèle LVDV-E équipé de système à cylindre coaxial, à l'aide des mobiles n° 18 ou 31 selon les viscosités, dans une chambre de mesure à température contrôlée à l'aide d'un bain thermostaté. Ces mesures sont effectuées à 20 °C pour des solutions aqueuses à 10% d'extrait sec du composé visqueux, à différentes vitesses de rotation des mobiles de 10 à 100 rpm. Les valeurs de viscosité retenues sont les valeurs obtenues aux vitesses de rotation les plus élevées en intégrant les valeurs limites du couple torsion (80% maximum) de l'appareil.

Selon la présente invention, le composé visqueux est la carboxyméthylcellulose (CMC). Ce composé apporte une protection de la substance sensible dans des conditions de chaleur et de pression, ce qui le rend quasiment insoluble alors que remis en solution, il s'hydrate et redevient biodisponible.

Par « substance sensible », on entend toute substance qui est susceptible de perdre une partie ou la totalité de son activité lorsqu'elle est soumise à des températures et/ou à des pressions et/ou à une humidité relative élevées. Ces contraintes peuvent être rencontrées notamment lors de la préparation de compositions contenant ladite substance, et plus particulièrement lors des étapes de granulation. Cette substance peut être notamment un mélange de substances sensibles.

Par « dégradation », on entend toute transformation de ladite substance sensible qui entraîne une perte d'activité, partielle ou totale, que ce soit par dénaturation de ladite substance ou par changement des proportions dans une composition comportant plusieurs substances, certaines étant notamment volatiles (par exemple dans le cas d'une huile essentielle).

La substance sensible peut être notamment choisie parmi les protéines, notamment les enzymes, les vitamines, les bactéries, les levures, les anti-oxydants, les caroténoïdes, les huiles essentielles ou des substances pharmacologiquement actives telles que les antibiotiques.

A titre d'exemple, on peut citer pour les enzymes, la Superoxydismutase, pour les vitamines, la Riboflavine ou l'acide ascorbique, pour les bactéries, le lactobacillus, pour les levures, saccharomyces cerevisiae, pour les anti-oxydants, les polyphénols ou la vitamine E, pour les caroténoïdes, la lutéine, l'astaxanthine, le lycopène, la xanthophylle ou les carotènes, pour les huiles essentielles naturelles, celles issues de l'ail, du thym ou du romarin et pour les antibiotiques, l'amoxycicline ou la tylosine.

Cette substance sensible peut être utilisée en particulier dans le domaine de l'alimentation animale ou humaine, dans le domaine pharmaceutique ou vétérinaire, dans le domaine des détergents, des produits de nettoyage et de lavage, notamment des vêtements.

Par « composition pulvérulente », on désigne des poudres comportant des particules présentant une granulométrie moyenne de 30 à 3 000 µm en moyenne.

Les particules de la composition pulvérulente comprennent deux parties bien distinctes : un coeur et un enrobage de ce coeur qui forme un film protecteur. Le coeur est constitué de la substance sensible et d'au moins un composé visqueux qui sert d'agent d'imprégnation. L'enrobage, quant à lui, contient au moins un composé visqueux qui sert d'agent d'engluage. Le composé visqueux intervient donc à deux niveaux puisqu'il est présent à la fois dans le coeur et l'enrobage. Il est possible d'utiliser deux composés visqueux différents, l'un contenu dans le coeur et l'autre dans l'enrobage.

Le composé visqueux utilisé en tant qu'agent d'imprégnation permet d'assurer un mélange homogène sans démélange des éléments constitutifs de la composition tout en liant tous les grains entre eux.

Le composé visqueux utilisé en tant qu'agent d'engluage permet, quant à lui, de réaliser la protection définitive. Ainsi, lors d'une étape de granulation, il assure d'une part, une protection contre les conditions d'humidité et de température élevées rencontrées lors de la mise en température de la composition à l'aide de vapeur d'eau lors du mélange avec l'aliment, et d'autre part, une protection contre les conditions de pression élevée et de cisaillement rencontrées lors du passage en filière.

Le coeur des particules de ladite composition pulvérulente contient au moins un support de ladite substance sensible.

En effet, le support par sa nature absorbante est une matière choisie comme étant déjà à l'état sec. Il protège la substance sensible par stabilisation de l'activité de l'eau lorsque le support est en contact avec la substance sensible sous forme liquide, pulvérisée lors de phases de séchage.

Ce support peut être en particulier de l'amidon, des farines, notamment de blé, de maïs, de manioc ou de riz, du talc, de la pulpe de betterave, de la maltodextrine, des sels tels que le carbonate de calcium ou des drèches de maïs.

Selon l'invention, l'agent d'imprégnation et l'agent d'engluage sont identiques.

Le composé visqueux a cependant des fonctions bien différentes s'il est utilisé en tant qu'agent d'imprégnation ou en tant qu'agent d'engluage, comme déjà énoncé ci-dessus, même s'il s'agit du même composé.

Selon l'invention, l'agent d'imprégnation et l'agent d'engluage sont constitués par la carboxyméthylcellulose (CMC).

Dans un mode de réalisation particulier, la substance sensible représente de 5 à 40%, préférentiellement de 10 à 30% d'extrait sec de la composition pulvérulente, le support représente de 10 à 60%, préférentiellement de 20 à 50% d'extrait sec de la composition pulvérulente, l'agent d'imprégnation représente de 5 à 40%, préférentiellement de 10 à 30% d'extrait sec de la composition pulvérulente et l'agent d'engluage représente de 5 à 30%, préférentiellement de 10 à 20% d'extrait sec de la composition pulvérulente.

Dans un autre mode de réalisation particulier, la substance sensible est constituée par un mélange d'enzymes constitué principalement de β-glucanase, de xylanase et de cellulase, le support est constitué par de la farine de blé, l'agent d'imprégnation et l'agent d'engluage sont constitués par la carboxyméthylcellulose (CMC).

La présente invention concerne également une composition pulvérulente stable contenant des particules comprenant :
- un coeur contenant au moins une substance sensible subissant moins de 20% de dégradation lorsque ladite composition pulvérulente stable est soumise à des températures de 60 °C à 100 °C et/ou à des pressions de 2.10⁶ à 10⁷ Pa et/ou à une humidité relative de 60% à 100% pendant l'étape de granulation de ladite composition, au moins un composé visqueux dont la viscosité est supérieure à 5 Pa.s en tant qu'agent d'imprégnation de la substance sensible et au moins un support de la substance sensible, et
- un enrobage du coeur, lequel enrobage contient au moins un composé visqueux dont la viscosité est supérieure à 5 Pa.s en tant qu'agent d'engluage,
dans laquelle la substance sensible n'est pas une enzyme destinée à l'alimentation d'animaux non humains,
lesdits composés visqueux étant choisis parmi la carboxyméthylcellulose (CMC).

Par « composition pulvérulente stable », on désigne des compositions dans lesquelles la substance sensible subit moins de 20% de dégradation lorsque ladite composition pulvérulente est soumise à des températures comprises de 60 °C à 100 °C et/ou à des pressions comprises de 2.10⁶ à 10⁷ Pa et/ou à une humidité relative de 60% à 100%.

La substance sensible peut être notamment choisie parmi les protéines les vitamines, les bactéries, les levures, les anti-oxydants, les caroténoïdes, les huiles essentielles ou des substances pharmacologiquement actives telles que les antibiotiques.

Le coeur contient au moins un support de la substance sensible.

Ce support peut être choisi en particulier parmi l'amidon, les farines, notamment de blé, de maïs, de manioc ou de riz, le talc, la pulpe de betterave, la maltodextrine, les sels tels que le carbonate de calcium ou les drèches de maïs.

Dans un mode de réalisation particulier, la composition pulvérulente est constituée de : 5 à 40%, préférentiellement 10 à 30% en extrait sec d'une substance sensible, 10 à 60%, préférentiellement 20 à 50% en extrait sec d'un support, 5 à 40%, préférentiellement 10 à 30% en extrait sec d'un agent d'imprégnation et 5 à 30%, préférentiellement 10 à 20% en extrait sec d'un agent d'engluage.

La présente invention concerne également un procédé de préparation d'une composition pulvérulente stable contenant des particules comprenant :
- un coeur contenant au moins une substance sensible subissant moins de 20% de dégradation lorsque ladite composition pulvérulente stable est soumise à des températures de 60 °C à 100 °C et/ou à des pressions de 2.10⁶ à 10⁷ Pa et/ou à une humidité relative de 60% à 100% pendant l'étape de granulation de ladite composition, au moins un composé visqueux dont la viscosité est supérieure à 5 Pa.s en tant qu'agent d'imprégnation de la substance sensible et au moins un support de la substance sensible, et
- un enrobage du coeur, lequel enrobage contient au moins un composé visqueux dont la viscosité est supérieure à 5 Pa.s en tant qu'agent d'engluage
lesdits composés visqueux étant choisis parmi la carboxyméthylcellulose (CMC),
ledit procédé comprenant :
une étape de microgranulation d'un mélange de ladite substance sensible et dudit agent d'imprégnation pour obtenir le coeur, et
une étape d'enrobage dudit coeur par ledit agent d'engluage.

Lors de ce procédé de préparation de la composition pulvérulente stable, le coeur des particules de cette composition pulvérulente est obtenu à partir d'un mélange de trois ingrédients, la substance sensible, un support et l'agent d'imprégnation, par une étape de microgranulation. Le coeur des particules est alors enrobé avec l'agent d'engluage pour former un film protecteur autour de la particule et ainsi protéger la substance sensible des futures contraintes qu'elle devra subir, notamment lors de la formation de granulés dans les presses à granuler. Cela permet également de la préserver lors du transport ou du stockage.

La combinaison du choix de l'agent d'imprégnation et de l'agent d'engluage, ainsi que de la technologie mise en oeuvre fait que la composition pulvérulente stable obtenue se compose de particules engluées d'un film protecteur lui conférant des propriétés nouvelles. En particulier, la composition pulvérulente a des propriétés hydrophobes et la substance sensible contenue dans ladite composition devient résistante à la température et à la pression et reste stable lors du transport ou du stockage. Cependant, la composition pulvérulente s'hydrate à nouveau et redevient donc biodisponible lorsqu'elle est remise en solution.

L'étape de microgranulation pourra être effectuée à partir d'un mélange de ladite substance sensible et dudit support préalablement co-séchés et de la totalité de l'agent d'imprégnation.

Lors d'un tel procédé, la substance sensible et le support préalablement co-séchés se présente sous forme d'un produit totalement homogène, avec une taille de particules et une dispersion maîtrisées, apte à être mélangé avec l'agent d'imprégnation

L'étape de microgranulation pourra également être effectuée à partir d'un mélange de ladite substance sensible, dudit support et de la totalité de l'agent d'imprégnation préalablement co-séchés.

Cette méthode permet avantageusement de disposer en direct d'un produit parfaitement homogène tout en réduisant une étape coûteuse de mélange.

L'étape de microgranulation pourra encore être effectuée à partir d'un mélange de ladite substance sensible, dudit support et d'une partie de l'agent d'imprégnation préalablement co-séchés et du reste de l'agent d'imprégnation.

Cette méthode donne une poudre intermédiaire, dont les tailles de particules sont contrôlées avec une dispersion réduite. La majeure partie de l'agent d'imprégnation est déjà présente, il suffit alors de compléter avec le reste de l'agent d'imprégnation pour démarrer la phase suivante.

Dans un mode de réalisation particulier, ledit procédé comprend une étape de co-séchage, avant l'étape de microgranulation, d'un mélange contenant la substance sensible, le support de la substance sensible et éventuellement tout ou partie de l'agent d'imprégnation, le co-séchage étant effectué à une température d'air de sortie inférieure à 60 °C et à une température de poudre inférieure à 45 °C.

Dans un mode de réalisation préféré du procédé de l'invention, la substance sensible sera utilisée sous forme liquide et le support sous forme sèche.

Dans un autre mode de réalisation particulier, ledit procédé comprend :
- **une étape de co-séchage** d'un mélange contenant la substance sensible et au moins un support de la substance sensible, le co-séchage étant effectué à une température d'air de sortie inférieure à 60 °C et à une température de poudre inférieure à 45 °C, pour obtenir une poudre homogène intermédiaire,
- **une étape de microgranulation** de la poudre homogène intermédiaire avec l'agent d'imprégnation pour obtenir une poudre microgranulée densifiée constituée de particules non enrobées correspondant au susdit coeur,

Dans un mode de réalisation préféré du procédé de l'invention, la substance sensible sera utilisée sous forme liquide, le support sous forme sèche et l'agent d'imprégnation sous forme solide.

L'étape de co-séchage est réalisée à basse température ce qui permet une rétention à plus de 95% de la substance sensible et conduit à l'obtention d'une poudre homogène dont la granulométrie moyenne mesurée par le D(v,o,5) peut être réglée de 50 à 250 µm.

La poudre homogène intermédiaire contient alors de 10 à 100%, notamment de 40 à 65%, en particulier 55% de substance sensible en extrait sec.

L'humidité finale de la poudre homogène intermédiaire varie de 0% à notamment de 5 à 12% et l'activité d'eau est inférieure à 0,6 pour éviter tout développement de microorganismes.

L'étape de microgranulation est généralement réalisée à une température maximale de 45 °C pour ne pas détériorer la substance sensible et à une teneur en eau donnée, soit environ de 5 à 20% d'eau rajouté par extrait sec total mis en oeuvre et préférentiellement 10%, pour que l'imprégnation et la consistance du produit soit atteinte, et ce en contrôlant l'homogénéité particulaire et en suivant le taux d'humidité et par un contrôle visuel à l'aide d'une loupe binoculaire ou par analyse d'image.

La poudre densifiée est caractérisée en ce qu'on observe un changement d'état du produit, au départ sous forme de poudre, du fait de l'agrégation des particules les unes avec les autres, et ce, juste avant d'obtenir la consistance d'un produit pâteux, ce qui est surveillé soit par la température qui s'accroît de manière très rapide, soit par la prise d'ampérage du moteur principal du microgranulateur .

La poudre densifiée obtenue a alors une granulométrie moyenne mesurée par le D(v,o,5) de 100 à 200 µm et une teneur en eau de 5 à 10%.

Cette poudre densifiée est constituée de 5 à 50%, de préférence de 10 à 35%, de substance sensible en extrait sec, de 10 à 70%, de préférence de 20 à 60%, de support en extrait sec et de 5 à 50%, de préférence de 10 à 30%, d'agent d'imprégnation.

Dans un mode de réalisation particulier, le procédé de l'invention de préparation de la composition pulvérulente comprend :
- **une étape de co-séchage** d'un mélange contenant la substance sensible et au moins un support de la substance sensible, le co-séchage étant effectué à une température d'air de sortie inférieure à 60 °C et à une température de poudre inférieure à 45 °C, pour obtenir une poudre homogène intermédiaire,
- **une étape de microgranulation** de la poudre homogène intermédiaire avec l'agent d'imprégnation pour obtenir une poudre microgranulée densifiée constituée de particules non enrobées correspondant au susdit coeur,
- **une étape d'enrobage** des particules non enrobées de la poudre microgranulée densifiée avec l'agent d'engluage pour obtenir une poudre enrobée.

Dans un mode de réalisation préféré du procédé de l'invention, la substance sensible sera utilisée sous forme liquide, le support sous forme sèche, l'agent d'imprégnation sous forme solide et l'agent d'engluage sous forme liquide, notamment en solution dans l'eau.

Lors de l'étape d'enrobage, le taux de dépôt d'agent d'engluage autour du coeur des particules dépend de la granulométrie finale souhaitée de la composition pulvérulente stable. Il varie notamment de 5 à 30%, de préférence de 10 à 20% d'extrait sec de la composition pulvérulente.

L'agent d'engluage est déposé notamment sous forme liquide, à partir d'une solution de 4 à 10% en extrait sec dans l'eau. La solution contenant l'agent d'engluage est telle qu'elle présente une viscosité compatible avec une pulvérisation permettant l'enrobage de la poudre densifiée grain à grain, sans agrégation des particules entre elles. ,

La poudre enrobée obtenue a une granulométrie moyenne mesurée par le D(v,o,5) de 300 à 400 µm et une teneur en eau de 5 à 12%.

Cette poudre enrobée est constituée de 5 à 40%, de préférence de 10 à 30%, de substance sensible en extrait sec, de 10 à 60%, de préférence de 20 à 50%, de support en extrait sec, de 5 à 40%, de préférence de 10 à 30%, d'agent d'imprégnation en extrait sec et de 5 à 30%, de préférence de 10 à 20%, d'agent d'engluage en extrait sec.

Dans un autre mode de réalisation particulier de l'invention, le procédé comprend :
- **une étape de co-séchage** d'un mélange contenant la substance sensible et au moins un support de la substance sensible, le co-séchage étant effectué à une température d'air de sortie inférieure à 60 °C et à une température de poudre inférieure à 45 °C, pour obtenir une poudre homogène intermédiaire,
- **une étape de microgranulation** de la poudre homogène intermédiaire avec l'agent d'imprégnation pour obtenir une poudre microgranulée densifiée constituée de particules non enrobées correspondant au susdit coeur,
- éventuellement **une étape de séchage à basse température** de la poudre microgranulée densifiée à une température inférieure à 45°C, pour obtenir une poudre microgranulée densifiée séchée,
- éventuellement **une étape de tamisage** pour obtenir une poudre microgranulée densifiée tamisée,
- **une étape d'enrobage** des particules non enrobées de la poudre microgranulée densifiée éventuellement séchée et éventuellement tamisée, avec l'agent d'engluage pour obtenir une poudre enrobée,
- **une étape de séchage** de la poudre enrobée pour obtenir une composition pulvérulente stable.

Par « séchage à basse température », on désigne une étape de séchage où la température n'excède pas 45 °C.

Cette étape de séchage est effectuée après l'étape de microgranulation si la poudre densifiée obtenue après cette étape de microgranulation présente une teneur en eau supérieure à 10% et afin d'obtenir une teneur en eau inférieure à 9%, et de préférence inférieure à 7%.

L'étape de tamisage est effectuée après l'étape de microgranulation afin de disposer de particules de taille équivalente avant l'enrobage.

La poudre microgranulée densifiée éventuellement séchée et éventuellement tamisée obtenue avant l'étape d'enrobage a alors une granulométrie moyenne mesurée par le D(v,o,5) de 100 à 200 µm et une teneur en eau de 10% maximum.

L'étape de séchage effectuée après l'étape d'enrobage est réalisée à basse température, c'est-à-dire à une température maximum de 45 °C à coeur, dans des conditions respectant la substance sensible et permet d'obtenir une composition pulvérulente stable ayant une teneur en eau inférieure à 10%, et de préférence inférieure à 8%.

La granulométrie de la composition pulvérulente stable est principalement comprise dans la gamme de 100 à 500 µm.

La composition pulvérulente stable ainsi obtenue peut être directement commercialisée comme matière première pour être introduite notamment dans des granulés à des fins notamment d'alimentation animale, car cette taille particulaire est celle qui évite tout démélange dans les produits formulés destinés à l'alimentation animale.

Selon l'invention, le composé visqueux est la carboxyméthylcellulose (CMC).

La substance sensible peut être notamment choisie parmi les protéines, notamment les enzymes, les vitamines, les bactéries, les levures, les anti-oxydants, les caroténoïdes, les huiles essentielles ou des substances pharmacologiquement actives telles que les antibiotiques.

Le support de la substance sensible peut être en particulier choisi parmi l'amidon, les farines, notamment de blé, de maïs, de manioc ou de riz, le talc, la pulpe de betterave, la maltodextrine, les sels tels que le carbonate de calcium, les drèches de maïs.

Selon l'invention, l'agent d'imprégnation et l'agent d'engluage sont identiques et constitués par la carboxyméthylcellulose (CMC).

Ce procédé est alors plus attractif économiquement d'une part parce que la CMC est peu coûteuse et d'autre part parce que ce procédé permet d'obtenir une résistance accrue à la température et à la pression pour un taux de CMC (utilisée en tant qu'agent d'imprégnation et d'engluage) pouvant être réduit à 35% voir jusqu'à 25% en extrait sec de composition pulvérulente finale (alors qu'il est classiquement de 55%).

Dans un mode de réalisation particulier, la substance sensible représente de 5 à 40%, préférentiellement de 10 à 30% d'extrait sec de la composition pulvérulente, le support représente de 10 à 60%, préférentiellement de 20 à 50% d'extrait sec de la composition pulvérulente, l'agent d'imprégnation représente de 5 à 40%, préférentiellement de 10 à 30% d'extrait sec de la composition pulvérulente et l'agent d'engluage représente de 5 à 30%, préférentiellement de 10% à 20% d'extrait sec de la composition pulvérulente.

La présente invention concerne également une composition pulvérulente obtenue par un procédé de l'invention tel que défini ci-dessus.

La présente invention concerne également une composition pharmaceutique comprenant, comme substance active, une composition pulvérulente stable contenant des particules comprenant :
- un coeur contenant au moins une substance sensible subissant moins de 20% de dégradation lorsque ladite composition pulvérulente stable est soumise à des températures de 60 °C à 100 °C et/ou à des pressions de 2.10⁶ à 10⁷ Pa et/ou à une humidité relative de 60% à 100% pendant l'étape de granulation de ladite composition, au moins un composé visqueux dont la viscosité est supérieure à 5 Pa.s en tant qu'agent d'imprégnation de la substance sensible, et au moins un support de la substance sensible, et
- un enrobage du coeur, lequel enrobage contient au moins un composé visqueux dont la viscosité est supérieure à 5 Pa.s en tant qu'agent d'engluage, dans laquelle la substance sensible possède au moins 80%, notamment au moins 90% de son activité initiale, et lesdits composés visqueux étant choisis parmi la carboxyméthylcellulose (CMC), en association avec un véhicule pharmaceutiquement acceptable.

Cette composition pharmaceutique peut notamment être destinée à l'animal.

La présente invention concerne également une composition alimentaire comprenant une composition pulvérulente stable telle que définie dans la présente invention, dans laquelle la substance sensible possède au moins 80%, notamment au moins 90% de son activité initiale, et n'est pas une enzyme destinée à l'alimentation d'animaux non humains, et au moins un produit alimentaire.

Cette composition alimentaire peut notamment être destinée à l'animal.

Cette composition alimentaire peut de plus être caractérisée en ce qu'elle se présente sous la forme de pellets ou de granulés.

La présente invention concerne également un procédé de préparation de compositions alimentaires comprenant une composition pulvérulente stable telle que définie dans la présente invention, et au moins un produit alimentaire, ledit procédé comprenant une étape de compression humide, notamment à 14% d'humidité, avec une pression appliquée de 2.10⁶ à 10⁷ Pa, d'un mélange de la composition pulvérulente stable avec le produit alimentaire.

Dans un mode de réalisation particulier, l'étape de co-séchage mentionnée ci-dessus est réalisée en continu, notamment sur une tour de séchage par atomisation et/ou lit fluidisé.

La substance sensible est introduite, lors de cette étape, par des buses disposées autour de l'arrivée du support sec ou par une cane centrale d'injection, ou un mélangeur dynamique de type Shugi® ou Bepex® (commercialisés par la société HOSOKAWA) sur des tours industrielles.

L'étape de co-séchage peut être réalisée en particulier en continu par pulvérisation de la substance sensible sous forme d'un aérosol mono dispersé, directement sur la poudre support par un dispositif approprié permettant de ce fait une meilleure efficacité et l'obtention de particules parfaitement homogènes.

Dans un mode de réalisation particulier, l'étape de microgranulation mentionnée ci-dessus se fait par microgranulation humide et cisaillement, notamment sur lit fluidisé.

Cette étape consiste notamment en une imprégnation de la poudre homogène intermédiaire avec l'agent d'imprégnation et une densification en discontinu en mélangeur statique ou en continu, par exemple dans un mélangeur dynamique SHUGI® (commercialisé par la société HOSOKAWA) par un dispositif rotatif comme un mélangeur rapide pour produits humides et pâteux (cutter) ou un granulateur humide utilisée en pharmacie DIOSNA®, TURBOSPHERE® ou GLATT® (commercialisés par les sociétés DIOSNA, Pierre GUERIN et GLATT, respectivement) équipé d'un système de pulvérisation adapté.

Dans un mode de réalisation préféré de l'invention, l'étape de microgranulation mentionnée ci-dessus est réalisée en continu par mouillage avec un aérosol monodispersé, permettant de ce fait une meilleure efficacité et l'obtention de particules homogènes.

Dans un mode de réalisation particulier, l'étape de séchage à basse température mentionnée ci-dessus est réalisée soit en lit fluidisé discontinu, soit directement si l'étape d'imprégnation s'est faite en continu, soit sur tour de séchage en utilisant les dispositifs de co-séchage par tube ou shugi.

Dans un mode de réalisation particulier, l'étape d'enrobage mentionnée ci-dessus consiste à déposer un film suffisant d'agent d'engluage, puis de sécher le produit obtenu. Le film est habituellement déposé par des buses de pulvérisation adaptées sur des sécheurs à lit d'air fluidisé.

Dans un mode de réalisation préféré de l'invention, l'étape d'enrobage par pulvérisation d'un film est réalisée en continu avec un aérosol mono dispersé, permettant de ce fait une meilleure efficacité et l'obtention de particules homogènes afin d'améliorer la densité des particules et limiter la quantité d'agent d'engluage déposée.

Dans un mode de réalisation préféré de l'invention, des systèmes novateurs de confinement de poudre d'imprégnation développés sur matériel INNOJET® (commercialisé par la société INNOJET) avec des buses ROTOJET® (commercialisées par la société INNOJET) peuvent être utilisés afin d'améliorer la densité des particules et limiter la quantité d'agent d'engluage déposée. Ce système permet une homogénéité du passage des particules devant la buse avec un débit élevé.

La poudre enrobée est alors séchée par lots successifs sur un lit fluidisé classique GLATT ou AEROMATIC bottom spray (commercialisés par les sociétés GLATT et GEA, respectivement), INNOJET®, top spray ou tangentiel, ou en continu avec un lit fluidisé continu avec une rampe de buses ou avec un agglomérateur SHUGI ou en lots (batch), le but étant de ramener la teneur en eau de la poudre enrobée à une valeur inférieure à 10% et préférentiellement inférieure à 8%.

### FIGURES

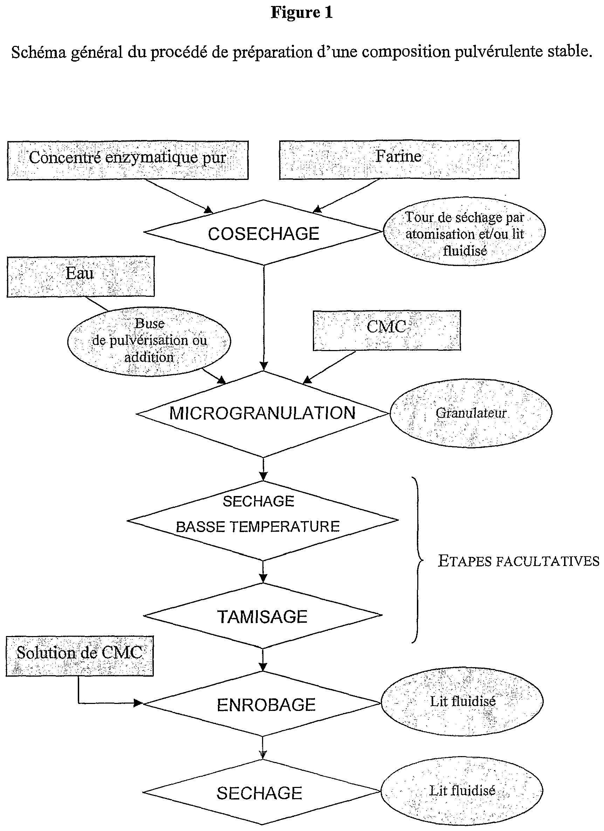

La Figure 1 présente de façon schématique les différentes étapes du procédé de préparation d'une composition pulvérulente stable selon l'invention, les matières premières étant indiquées dans un rectangle grisé, les opérations effectuées étant indiquées dans un losange blanc et le matériel et les paramètres utilisés étant indiqués dans un ovale grisé. Le code utilisé est illustré ci-dessous 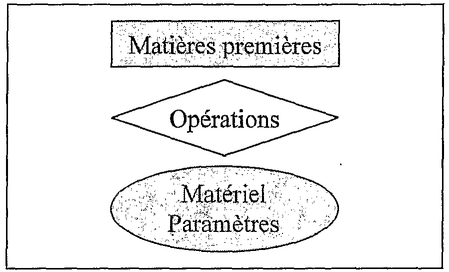
Les Figures 2 à 7 présentent différentes photos de particules d'une composition pulvérulente observée par microscope électronique à balayage.

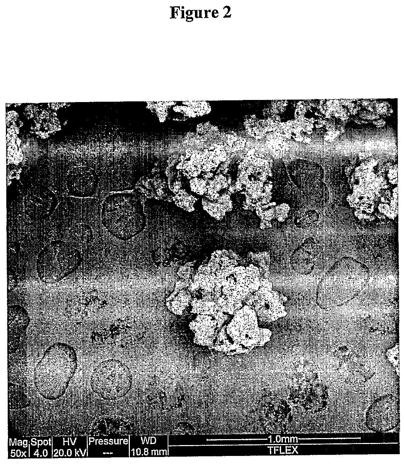

Les figures 2 (grossissement x50), 3 (grossissement x200) et 4 (grossissement x800) concernent des particules obtenues par une méthode d'enrobage classique sur lit fluidisé.

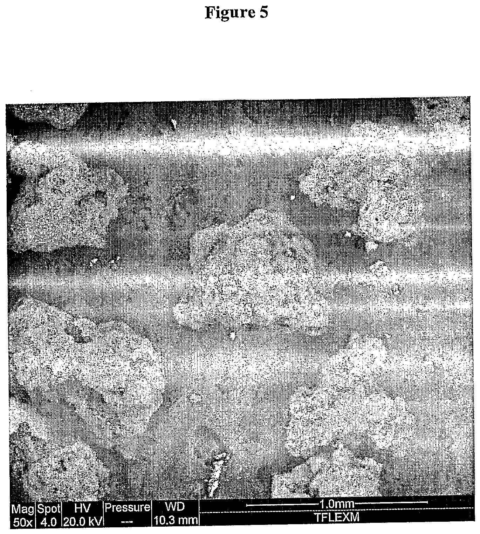

Les figures 5 (grossissement x50), 6 (grossissement x200) et 7 (grossissement x800) représentent des particules obtenues par le procédé de l'invention utilisant des systèmes novateurs d'imprégnation développés sur du matériel INNOJET®.

### EXEMPLES

### Exemple 1 : Préparation d'une composition pulvérulente stable contenant un mélange d'enzymes

Le protocole expérimental ci-dessous décrit un procédé de préparation d'une composition pulvérulente stable dans laquelle la substance sensible est un mélange d'enzymes, le support est de la farine et les agents d'imprégnation et d'engluage sont constitués par la CMC.

### 1. Etape de co-séchage

475 kg/h de solution enzymatique à 28,7% de matière sèche (MS) sont pulvérisés à 165.10⁵ Pa et co-séchés en continu sur un support de type farine, à raison de 113 kg/h, à basse température :
- température de l'air d'entrée : 146 °C
- température de l'air de sortie : 49 °C
- température de l'air du lit statique : 40 °C
- température de l'air du vibro fluidiseur (première section) : 35 °C
- température de l'air du vibro fluidiseur (deuxième section) : 27 °C

La répartition de l'extrait sec est donc la suivante : 55% d'un mélange d'enzymes et 45% de farine de blé utilisée comme support.

Le mélange d'enzymes est un moût de fermentation filtré concentré obtenu à partir de la fermentation de *Penicillium funiculosum* (IMI 378536) qui contient 19 activités enzymatiques dont les principales sont la cellulase, la xylanase et la β-glucanase.

Le *Penicillium funiculosum* utilisé est protégé par le brevet EP 1 007 743 et a été déposé le 24 Mars 1998 par la société ADISSEO sous le numéro IMI 378536 auprès de l'IMI (International Mycological Institute, Bakeham Lane, Englefield Green, Egham, Surrey, TW20 9TY, Grande-Bretagne), reconnu autorité de dépôt international selon le Traité de Budapest.

La poudre homogène obtenue à ce stade présente un caractère de poudre sans fines inférieures à 63 µm, ce qui en fait un produit apte à être manipulé dans une deuxième phase d'imprégnation de ladite poudre avec un agent d'imprégnation. La granulométrie moyenne en D(v,0,5) est de 117 µm et l'humidité de 7,5%.

A l'aide de ces dispositifs, une liaison forte est obtenue entre le support et le liquide contenant l'enzyme, même dans le cas de produits difficiles à granuler comme la farine de blé ou de maïs.

Cette étape permet d'obtenir une poudre homogène intermédiaire sèche pré-stabilisée.

### 2. Etape d'imprégnation avec l'agent d'imprégnation

L'imprégnation est effectuée dans un mélangeur rapide pour produits humides et pâteux (cutter) et comprend les étapes suivantes :
∘ 30 secondes de mélange préalable des ingrédients (0,77 kg d'extrait sec de CMC par kilogramme d'extrait sec de poudre homogène intermédiaire),
∘ 120 secondes d'addition d'eau, à raison de 0,18 kg d'eau par kilogramme d'extrait sec de poudre intermédiaire, sous agitation, et
∘ 120 secondes de mélange terminal.

L'objet à ce stade est d'obtenir des particules de l'ordre de 150 à 200 µm en moyenne, avec une humidité de 14%, tout en maintenant le produit à une température inférieure à 45 °C afin de ne pas dégrader la substance sensible.

Le produit est ainsi séché sur sécheur à lit fluidisé avec une température d'entrée de 55 °C et une température de sortie de 25 °C.

La poudre microgranulée densifiée obtenue présente de 7 à 8% d'humidité.

### 3. Etape d'enrobage avec l'agent d'engluage

Une solution aqueuse de 7 à 7,5% de CMC est réchauffée à une température comprise de 60 °C à 70 °C, avant d'être pulvérisée sur la poudre microgranulée densifiée. La quantité de CMC déposée est de 0,25 kg d'extrait sec de CMC par kilogramme d'extrait sec de poudre micro granulée densifiée à enrober.

La poudre obtenue à ce stade a une granulométrie moyenne de 300 à 400 µm et présente une humidité de 10 à 11%.

Le produit enrobé est alors séché en continu sur un lit fluidisé ou en lots (batch), le but étant de ramener le produit à une teneur en eau inférieure à 10% et préférentiellement inférieure à 8%.

La composition pulvérulente stable obtenue comme produit final possède alors la composition suivante : 25% en extrait sec d'enzymes, 20% en extrait sec de support, 55% en extrait sec de CMC (35% étant utilisé pour l'étape d'imprégnation et 20% étant utilisé pour l'étape d'enrobage).

La composition pulvérulente a par ailleurs une teneur en eau de 7,6%, une densité de 450 g/l et la granulométrie suivante :
∘ supérieure à 800 µm → 0%
∘ entre 500 et 800 µm → 12%
∘ entre 300 et 500 µm → 46%
∘ entre 200 et 300 µm → 26%
∘ entre 100 et 200 µm → 16%
∘ inférieure à 100 µm → 0%

### 4. Bilan

Les caractéristiques de la poudre obtenue à l'issue de chaque étape sont résumées dans le tableau 1 ci-dessous.

**Tableau 1**

| **Etape** | **Diamètre (en µm)** | **Taux d'humidité** |
|---|---|---|
| **Co-séchage** (poudre intermédiaire) | 117 en moyenne | 7,5% |
| **Imprégnation** (poudre densifiée) | 150-200 | - avant séchage : 14% |
| | | - après séchage : 7 à 8% |
| **Enrobage** (composition pulvérulente stable) | 300-400 | - avant séchage : 10 à 11% |
| | | - après séchage : < 10%, notamment < 8% |

### Exemple 2 : Préparation d'une composition pulvérulente stable contenant une enzyme superoxydismutase (SOD)

Le protocole expérimental ci-dessous décrit un procédé de préparation d'une composition pulvérulente stable dans laquelle la substance sensible est une enzyme superoxydismutase, le support est de la maltodextrine de blé et les agents d'imprégnation et d'engluage sont constitués par la CMC.

### 1. Etape de co-séchage

95 kg/h de solution enzymatique à 9,5% de matière sèche (MS) sont pulvérisés à 95.10⁵ Pa et co-séchés en continu sur un support de type maltodextrine, à raison de 9,5 kg/h, à basse température :
- température de l'air d'entrée : 110°C
- température de l'air de sortie : 50°C
- température de l'air du lit statique : 40 °C

La répartition de l'extrait sec est donc la suivante : 50% d'enzyme pur et 50% de support maltodextrine de blé.

La granulométrie moyenne de la poudre obtenue en D(v,0,5) est de 95 µm et l'humidité de 4,5%.

Cette étape permet d'obtenir une poudre homogène intermédiaire sèche pré-stabilisée.

### 2. Etape d'imprégnation avec l'agent d'imprégnation

L'imprégnation est effectuée dans un mélangeur rapide pour produits humides et pâteux (cutter) et comprend les étapes suivantes :
∘ 60 secondes de mélange préalable des ingrédients (0,25 kg d'extrait sec de CMC par kilogramme d'extrait sec de poudre intermédiaire),
∘ 90 secondes d'addition d'eau, à raison de 1 kg d'eau par kilogramme d'extrait sec de poudre intermédiaire, sous agitation, et
∘ 90 secondes de mélange terminal.

Le produit est séché sur sécheur à lit fluidisé avec une température d'entrée de 60 °C et une température de sortie de 40 °C.

La poudre microgranulée densifiée obtenue présente 8% d'humidité.

### 3. Etape d'enrobage avec l'agent d'engluage

Une solution aqueuse de 7 % de CMC est réchauffée à une température comprise de 60 °C à 70 °C. La quantité de CMC déposée est de 0,5 kg d'extrait sec de CMC par kilogramme d'extrait sec de poudre microgranulée densifiée à enrober.

La poudre obtenue à ce stade a une granulométrie moyenne de 300 µm et présente un taux d'humidité de 9%.

La composition pulvérulente stable obtenue comme produit final possède alors la composition suivante : 20% en extrait sec d'enzyme, 20% en extrait sec de support, 60% en extrait sec de CMC (10% étant utilisé pour l'étape d'imprégnation et 50% étant utilisé pour l'étape d'enrobage).

La composition pulvérulente a par ailleurs une teneur en eau de 9 %, une densité de 400 g/l et la granulométrie suivante :
∘ supérieure à 800 µm → 0%
∘ entre 500 et 800 µm → 7%
∘ entre 300 et 500 µm → 35%
∘ entre 200 et 300 µm → 38%
∘ entre 100 et 200 µm → 20%
∘ inférieure à 100 µm → 0%

### Exemple 3 : Préparation d'une composition pulvérulente stable contenant une bactérie

Le protocole expérimental ci-dessous décrit un procédé de préparation d'une composition pulvérulente stable dans laquelle la substance sensible est une bactérie de type lactobacillus, le support et l'agent d'imprégnation sont de l'amidon Perfectamyl® et l'agent d'engluage est constitué par la CMC.

### 1. Etape de co-séchage

105 kg/h d'un milieu de fermentation à 10% de matière sèche (MS) contenant 10⁹ bactéries par gramme sont pulvérisés à 50.10⁵ Pa et co-séchés en continu sur un support amidon Perfectamyl®, à raison de 45 kg/h, à basse température :
- température de l'air d'entrée : 90°C
- température de l'air de sortie : 50°C

La répartition de l'extrait sec est donc la suivante : 20% d'extrait sec de bactérie pur et 80% de support amidon.

La granulométrie moyenne de la poudre obtenue en D(v,0,5) est de 45 µm et l'humidité est de 4 %.

Cette étape permet d'obtenir une poudre homogène intermédiaire sèche pré-stabilisée.

### 2. Etape d'imprégnation avec l'agent d'imprégnation

L'imprégnation est effectuée dans un mélangeur rapide en atmosphère contrôlée et climatisée et comprend les étapes suivantes :
∘ 30 secondes de mélange préalable des ingrédients (0,2 kg d'extrait sec de CMC par kilogramme d'extrait sec de poudre intermédiaire)
∘ 30 secondes d'addition d'eau, à raison de 0,4 kg d'eau par kilogramme d'extrait sec de poudre intermédiaire, sous agitation, et
∘ 30 secondes de mélange terminal.

Le produit est séché sur sécheur à lit fluidisé avec un air d'entrée à moins de 2 g d'eau par kilogramme d'air, une température d'entrée de 50 °C et une température de sortie de 40 °C maximum.

La poudre microgranulée densifiée obtenue présente 7% d'humidité.

### 3. Etape d'enrobage avec l'agent d'engluage

Une solution aqueuse de 7 % de CMC est réchauffée à une température comprise de 60 °C à 70 °C. La quantité de CMC déposée est de 0,66 kg d'extrait sec de CMC par kilogramme d'extrait sec de poudre microgranulée densifiée à enrober.

La poudre obtenue à ce stade a une granulométrie moyenne de 350 µm et présente une humidité de 7 % et une activité d'eau inférieur à 0,2 afin de stabiliser les bactéries à coeur.

La composition pulvérulente stable obtenue comme produit final possède alors la composition suivante : 10% en extrait sec du milieu pur de fermentation de bactéries, 50% en extrait sec d'amidon (40% étant utilisé comme support et 10 % comme agent d'imprégnation) et enfin 40% en extrait sec de CMC utilisée comme agent d'engluage.

La composition pulvérulente a par ailleurs une teneur en eau de 7 % et une densité de 450 g/l.

### Exemple 4 : Préparation d'une composition pulvérulente stable contenant un antibiotique

Le protocole expérimental ci-dessous décrit un procédé de préparation d'une composition pulvérulente stable dans laquelle la substance sensible est un antibiotique, qui est la tylosine, déjà sous forme poudre, le support est de la farine et l'agent d'imprégnation et l'agent d'engluage sont constitués par de la CMC.

### 1. Etape de co-mélange

10 kg d'un antibiotique tylosine sous forme de poudre à 96,4% (MS) sont mélangés avec 13 kg de farine de blé.

La répartition de l'extrait sec est donc la suivante : 44% d'extrait sec d'antibiotique pur et 56% de farine.

La granulométrie moyenne de la poudre obtenue en D(v,0,5) est de 40 µm et l'humidité de 4,7%.

Cette étape permet d'obtenir une poudre homogène intermédiaire sèche pré-stabilisée en activité d'eau.

### 2. Etape d'imprégnation avec l'agent d'imprégnation

L'imprégnation est effectuée dans un mélangeur rapide en atmosphère contrôlée et climatisée et comprend les étapes suivantes :
∘ 90 secondes de mélange préalable des ingrédients (0,4 kg d'extrait sec de CMC par kilogramme d'extrait sec de poudre intermédiaire)
∘ 60 secondes d'addition d'eau, à raison de 0,1 kg d'eau par kilogramme d'extrait sec de poudre intermédiaire, sous agitation, et
∘ 60 secondes de mélange terminal.

Le produit est séché sur sécheur à lit fluidisé avec un air d'entrée à moins de 2 g d'eau par kilogramme d'air, une température d'entrée de 60 °C et une température de sortie de 50 °C maximum.

La poudre microgranulée densifiée obtenue présente 7 % d'humidité

### 3. Etape d'enrobage avec l'agent d'engluage

Une solution aqueuse de 7 % de CMC est réchauffée à une température comprise de 60 °C à 70 °C. La quantité de CMC déposée est de 0,14 kg d'extrait sec de CMC par kilogramme d'extrait sec de poudre microgranulée densifiée à enrober.

La poudre obtenue à ce stade a une granulométrie moyenne de 500 µm et présente une humidité de 5,6% et une activité d'eau inférieure à 0,2 afin de stabiliser l'antibiotique dans les mélanges lors de l'application.

La composition pulvérulente stable obtenue comme produit final possède alors la composition suivante : 27,5% en extrait sec d'antibiotique, 35% en extrait sec de support, 37,5% en extrait sec de CMC (25% étant utilisé pour l'étape d'imprégnation et 12,5% étant utilisé pour l'étape d'enrobage).

La composition pulvérulente stable a par ailleurs une densité de 450 g/l.

### Exemple 5 : Préparation d'une composition pulvérulente stable contenant un mélange de caroténoïdes

Le protocole expérimental ci-dessous décrit un procédé de préparation d'une composition pulvérulente stable dans laquelle la substance sensible est un mélange de caroténoïdes contenant notamment 55% d'astaxanthine, le support est du carbonate de calcium et les agents d'imprégnation et d'engluage sont constitués par la CMC.

### 1. Etape de co-séchage

400 kg/h d'un milieu de fermentation riche en astaxanthine à 15% de matière sèche (MS) sont pulvérisés à 100.10⁵ Pa et co-séchés en continu sur un support de type carbonate de calcium, à raison de 20 kg/h, à basse température :
- température de l'air d'entrée : 130°C
- température de l'air de sortie : 50°C
- température de l'air du lit statique : 40 °C

La répartition de l'extrait sec est donc la suivante : 75% de matière sèche de la solution de caroténoïdes et 25% de support carbonate de calcium.

La granulométrie moyenne de la poudre obtenue en D(v,0,5) est de 105 µm et l'humidité est de 5%.

Cette étape permet d'obtenir une poudre homogène intermédiaire sèche pré-stabilisée.

### 2. Etape d'imprégnation avec l'agent d'imprégnation

L'imprégnation est effectuée dans un mélangeur rapide pour produits humides et pâteux (cutter) et comprend les étapes suivantes :
∘ 90 secondes de mélange préalable des ingrédients (0,5 kg d'extrait sec de CMC par kilogramme d'extrait sec de poudre intermédiaire),
∘ 90 secondes d'addition d'eau, à raison de 1 kg d'eau par kilogramme d'extrait sec de poudre intermédiaire, sous agitation, et
∘ 180 secondes de mélange terminal.

Le produit est séché sur sécheur à lit fluidisé avec une température d'entrée de 70 °C et une température de sortie de 50 °C.

La poudre microgranulée densifiée obtenue présente 8% d'humidité.

### 3. Etape d'enrobage avec l'agent d'engluage

Une solution aqueuse de 7 % de CMC est réchauffée à une température comprise de 60 °C à 70 °C. La quantité de CMC déposée est de 0,25 kg d'extrait sec de CMC par kilogramme d'extrait sec de poudre microgranulée densifiée à enrober.

La poudre obtenue à ce stade a une granulométrie moyenne de 300 µm et présente une humidité de 8%.

La composition pulvérulente stable obtenue comme produit final possède alors la composition suivante : 40% en extrait sec de la solution de caroténoïdes, 13% en extrait sec de carbonate de calcium en tant que support, 47% en extrait sec de CMC (27% étant utilisé pour l'étape d'imprégnation et 20% étant utilisé pour l'étape d'enrobage).

La composition pulvérulente stable a par ailleurs une teneur en eau de 8 % et une densité de 510 g/l.

### Exemple 6 : Préparation d'une composition pulvérulente stable contenant une huile essentielle

Le protocole expérimental ci-dessous décrit un procédé de préparation d'une composition pulvérulente stable dans laquelle la substance sensible est une huile essentielle, le support est de la farine de blé, l'agent d'imprégnation et l'agent d'engluage sont constitués par la CMC.

### 1. Etape de co-mélange

50 kg/h d'une huile essentielle d'ail sont pulvérisés à 150.10⁵ Pa et co-mélangés en continu sur un support de type farine de blé, à raison de 54 kg/h, à basse température :
- température de l'air d'entrée : 20°C

La répartition de l'extrait sec est donc la suivante : 50% de matière sèche de l'huile essentielle d'ail et 50% de support farine de blé.

La granulométrie moyenne de la poudre obtenue en D(v,0,5) est de 50 µm et l'humidité de 7%.

Cette étape permet d'obtenir une poudre homogène intermédiaire pré-stabilisée.

### 2. Etape d'imprégnation avec l'agent d'imprégnation

L'imprégnation est effectuée dans un mélangeur rapide pour produits humide et pâteux (cutter) et comprend les étapes suivantes :
∘ 120 secondes de mélange préalable des ingrédients (0,15 kg d'extrait sec de CMC par kilogramme d'extrait sec de poudre intermédiaire),
∘ 90 secondes d'addition d'eau, à raison de 1 kg d'eau par kilogramme d'extrait sec de poudre intermédiaire, sous agitation, et
∘ 180 secondes de mélange terminal.

Le produit est séché sur sécheur à lit fluidisé avec une température d'entrée de 50 °C et une température de sortie de 40 °C.

La poudre microgranulée densifiée obtenue présente 7% d'humidité.

### 3. Etape d'enrobage avec l'agent d'engluage

Une solution aqueuse de 7 % de CMC est réchauffée à une température comprise de 60 °C à 70 °C. La quantité de CMC déposée est de 0,18 kg d'extrait sec de CMC par kilogramme d'extrait sec de poudre microgranulée densifiée à enrober.

La poudre obtenue à ce stade a une granulométrie moyenne de 450 µm et présente une humidité de 8%.

La composition pulvérulente stable obtenue comme produit final possède alors la composition suivante : 37,5% en extrait sec d'huile essentielle d'ail, 37,5% en extrait sec de farine de blé en tant que support, 25% en extrait sec de CMC (10% étant utilisé pour l'étape d'imprégnation et 15 % étant utilisé pour l'étape d'enrobage).

La composition pulvérulente stable a par ailleurs une teneur en eau de 8 % et une densité de 550 g/l.

### Exemple 7 : Caractérisation microscopique de la composition pulvérulente stable contenant un mélange d'enzymes

### 1. Protocole Expérimental

Deux types de compositions pulvérulentes stables ont été observés au microscope électronique à balayage :
- une composition pulvérulente obtenue avec un procédé d'enrobage classique de type GLATT® ou AEROMATIC®, contenant 55% de CMC en pourcentage de l'extrait sec, et
- une composition pulvérulente obtenue avec un procédé d'enrobage sur des systèmes novateurs d'imprégnation développés sur du matériel INNOJET®, contenant 35% de CMC en pourcentage de l'extrait sec, avec les buses ROTOJET®.

La méthode d'enrobage classique consiste à recouvrir un support solide à l'aide d'une couche de produit en mettant en suspension le matériau à enrober dans un courant d'air et en pulvérisant le liquide d'enrobage dans le lit fluidisé constitué.

La méthode d'enrobage utilisant des systèmes novateurs consiste, quant à elle, à pulvériser l'agent d'engluage, pour former un film protecteur, en continu avec un aérosol mono dispersé, permettant de ce fait une meilleure efficacité et l'obtention de particules homogènes afin d'améliorer la densité des particules et limiter la quantité d'agent d'engluage déposée. Ce système permet une homogénéité du passage des particules devant la buse avec un débit élevé.

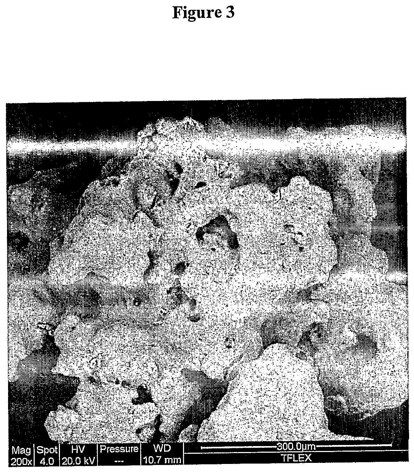

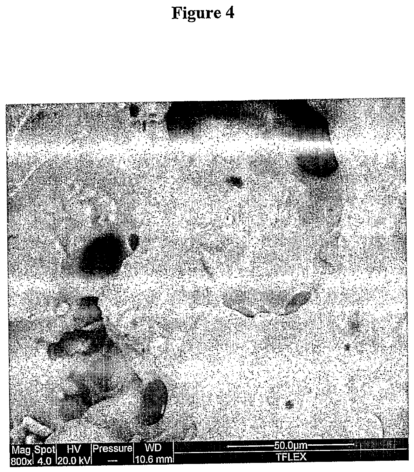

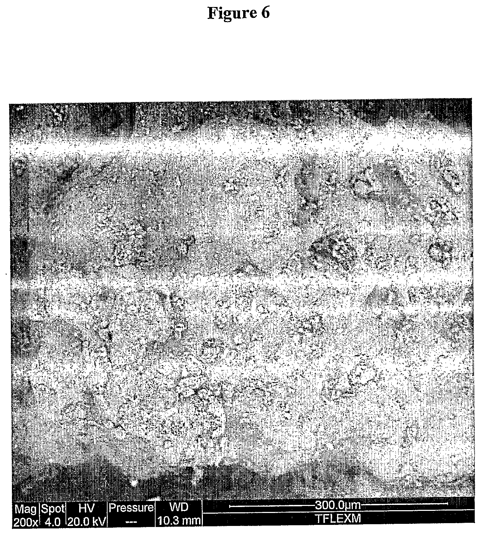

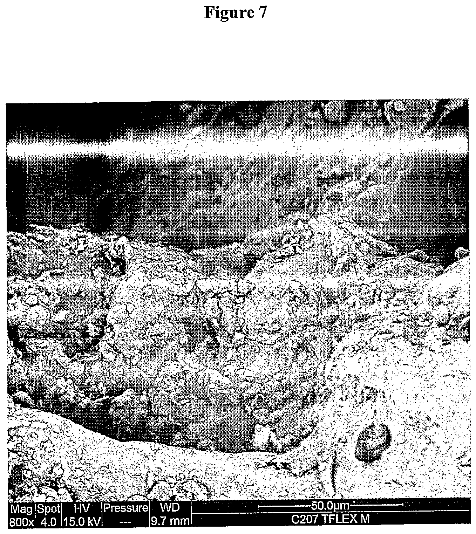

On observe ainsi que le dépôt de l'agent d'engluage effectué avec la méthode novatrice (voir Figures 5 à 7)est plus régulier, compact et densifié qu'avec une méthode d'enrobage classique (voir Figures 2 à 4), permettant de ce fait de réduire la quantité de CMC utilisée de manière avantageuse.

### 2. Résultats

Le film protecteur (ou enrobage) recouvrant le coeur des particules est visible au microscope électronique à balayage.

Dans le cas d'un procédé d'enrobage classique, on constate une hétérogénéité des particules avec la présence de vides partiels d'où une difficulté à déposer l'agent d'engluage dans les vides créés (voir Figures 2, 3 et 4).

Les particules obtenues par un procédé d'enrobage sur des systèmes novateurs d'imprégnation, qui contiennent donc moins de CMC, sont plus compactes et denses (voir Figures 5, 6 et 7).

### Exemple 8 : Résistance à la température et à la pression des enzymes contenues dans la composition pulvérulente stable

### 1. Protocole expérimental de préparation de granulés

L'objectif est de tester l'activité enzymatique de préparations constituées de compositions pulvérulentes contenant des enzymes ajoutées à une base d'aliment farine volaille croissance après une étape de granulation.

Trois températures de traitement en sortie de conditionneur (80, 85 et 90°C) sont appliquées pour chaque mélange suivant un protocole décrit ci-dessous.

### 1.1. Préparations contenant les enzymes

Test 1 : Un mélange de 60 kg réalisé avec 59,998 kg d'aliment farine volaille croissance et 3 g d'une composition pulvérulente est préparé à l'aide d'une mélangeuse à pâles à axe horizontal tournant à 60 tr/min. La durée du mélange est de 2 minutes.

Test 2 : Un prémélange de 500 g est réalisé avec 2 g d'une composition pulvérulente et 498 gd'aliment farine volaille croissance.

Un mélange de 40 kg réalisé avec 39,500 kg d'aliment farine volaille croissance et le prémélange précédent est préparé à l'aide d'une mélangeuse à pâles à axe horizontal tournant à 60 tr/min. La durée du mélange est de 2 minutes.

La composition pulvérulente utilisée consiste alors en :
- une composition standard sans protection pour les produits A et C correspondant au produit ROVABIO EXCEL AP commercialisé par ADISSEO France SAS, qui contient 80% de farine de blé et 20% d'une substance sensible, et
- une composition selon l'invention avec protection contenant 20% d'une substance sensible, 25% de support farine de blé et 55% de CMC (utilisée en tant qu'agent d'imprégnation et agent d'engluage) pour le produit B ou 35% de CMC pour les produits D et E,
le taux d'incorporation de ladite composition pulvérulente à l'aliment farine volaille croissance étant de 50 g/T.

Ces différentes compositions ont été produites à partir du même lot de substance sensible, à savoir un moût de fermentation filtré concentré obtenu à partir de *Penicillium funiculosum* (IMI 378536), contenant 19 activités enzymatiques dont les principales sont la xylanase, la β-glucanase et la cellulase, et qui est à l'origine du produit commercial ROVABIO EXCEL AP.

Le mélange précédent (test 1 ou 2) est vidangé dans un bac rectangulaire avant sa mise en sacs. Un échantillon représentatif du mélange d'environ 1 kg est prélevé par regroupement de 20 échantillons obtenus par quartage dans le bac rectangulaire.

### 1.2. Etapes de granulation

Les essais de granulation sont réalisés sur une presse de laboratoire à filière plate (Presse KAHL 14-175 de 3 kW). La filière de presse utilisée a des canaux de 4 mm de diamètre et de 24 mm d'épaisseur (taux de compression: 6).

Pour chaque essai de granulation, un sécheur refroidisseur de laboratoire est utilisé pour sécher et refroidir les échantillons de granulés chauds recueillis en sortie de filière. Le temps de séchage refroidissement est au minimum de 5 minutes pour une charge en granulés chauds d'environ 3,5 kg par refroidisseur.

Un échantillon représentatif de chaque fabrication de granulés d'environ 500 g est prélevé.

### 1.3. Mesures et prélèvements destinés au contrôle de la granulation

Les mesures et les prélèvements sont réalisés pendant le régime stabilisé de la presse (Débit constant, Puissance Electrique Consommée et Températures stables).

Les mesures d'humidité et de temps de séjour en filière sont réalisées à partir d'échantillons prélevés pendant la phase stabilisée de chaque essai.

Les caractérisations et paramètres de conduite de pressage sont relevés et enregistrés pendant les essais (voir en annexes les Tableaux de bord de granulation).

### Mesure sur la vapeur :

Les valeurs de la pression et du débit de vapeur sont enregistrées chaque seconde par un logiciel d'acquisition.

L'ouverture de la vanne de régulation de la vapeur est relevée manuellement sur le boîtier de régulation.

### Mesure de la température et de l'humidité :

Les températures (air ambiant, préparation avant et après conditionneur, filière) sont enregistrées chaque seconde par un logiciel d'acquisition.

La température de la filière correspond à la température des granulés.

Les mesures d'humidité sont réalisées après séchage à l'étuve d'un échantillon de 5 g à 103 °C pendant 4 heures. Toutes les mesures sont réalisées en double.

### Mesure de la consigne du Bis-vis d'alimentation :

Cette mesure est relevée manuellement au cours de chaque essai.

### Mesure du débit de la presse :

La mesure du débit de la presse est réalisée par pesée d'un prélèvement de 30 secondes en sortie de filière.

### Mesure de la puissance électrique consommée :

Cette mesure calculée par un convertisseur de puissance est enregistrée chaque seconde par un logiciel d'acquisition.

Avec le débit, ce paramètre nous permet de calculer la production spécifique nette (kg/kWh) et la consommation spécifique nette (kWh/T).

### Mesure du temps de séjour des granulés dans la filière :

Cette mesure est calculée par rapport au débit de la presse.

Le calcul prend en compte la pesée de 20 cm de granulés.

Les paramètres de granulation sont repris dans les tableaux de synthèse suivants et représentés de façon synthétique pour chaque phase de transformation du produit.

**Tableau 2 : Préparation à l'entrée du conditionneur**

| Essais | Température de traitement | Alimentation Bis-vis (mV)* | Température (°C)** | Humidité (%)*** |
|---|---|---|---|---|
| A | 80 °C | 37 | 18,6 | 10,7 |
| B | | 37 | 18,8 | 10,9 |
| C | | 37 | 17,8 | 10,1 |
| D | | 37 | 17,9 | 10,0 |
| E | | 37 | 17,9 | 10,2 |
| A | 85 °C | 38 | 19,2 | 10,7 |
| B | | 37 | 19,2 | 10,9 |
| C | | 36 | 18,3 | 10,1 |
| D | | 36 | 18,4 | 10,0 |
| E | | 36 | 18,3 | 10,2 |
| A | 90 °C | 37 | 19,5 | 10,7 |
| B | | 37 | 19,6 | 10,9 |
| C | | 36 | 19,0 | 10,1 |
| D | | 36 | 18,9 | 10,0 |
| E | | 36 | 18,9 | 10,2 |

| | | | | |
|---|---|---|---|---|
| * valeur lue au cours de chaque essai ** moyenne sur l'enregistrement de l'essai (1 valeur toutes les secondes) *** moyenne sur deux mesures | | | | |

**Tableau 3 : Préparation à la sortie du conditionneur**

| Essais | Température de traitement | Température (°C)* | Pression de vapeur (10⁵ Pa)* | Débit de vapeur (kg/h)* | Humidité (%)** |
|---|---|---|---|---|---|
| A | 80 °C | 80,4 | 1,6 | 4,5 | 15,4 |
| B | | 80,2 | 1,6 | 4,5 | 15,2 |
| C | | 80,1 | 1,6 | 4,7 | 14,8 |
| D | | 80,1 | 1,6 | 4,7 | 14,7 |
| E | | 80,2 | 1,6 | 4,5 | 14,7 |
| A | 85 °C | 85,1 | 1,6 | 4,8 | 15,7 |
| B | | 85,4 | 1,6 | 4,8 | 15,5 |
| C | | 85,1 | 1,6 | 5,2 | 15,0 |
| D | | 85,3 | 1,6 | 5,0 | 14,8 |
| E | | 85,0 | 1,6 | 5,2 | 14,9 |
| A | 90 °C | 90,1 | 1,6 | 5,6 | 16,3 |
| B | | 90,4 | 1,6 | 5,6 | 15,8 |
| C | | 90,0 | 1,6 | 5,8 | 15,3 |
| D | | 90,2 | 1,6 | 5,5 | 15,2 |
| E | | 90,0 | 1,6 | 5,5 | 15,4 |

| | | | | | |
|---|---|---|---|---|---|
| * moyenne sur l'enregistrement de l'essai (1 valeur toutes les secondes) ** moyenne sur deux mesures | | | | | |

**Tableau 4 : Granulés en sortie de presse**

| Essais | Température de traitement | Température filière (°C)* | Ecart filière (°C)** | Temps de séjour filière (s)*** | Débit presse (kg/h)**** |
|---|---|---|---|---|---|
| A | 80 °C | 80,7 | 0,3 | 7,3 | 42,3 |
| B | | 80,7 | 0,5 | 7,6 | 40,6 |
| C | | 80,1 | 0,0 | 7,3 | 41,7 |
| D | | 80,8 | 0,7 | 7,4 | 41,5 |
| E | | 80,0 | 0,8 | 7,4 | 41,7 |
| A | 85 °C | 83,4 | -1,7 | 7,4 | 40,7 |
| B | | 83,4 | -2,0 | 7,3 | 42,0 |
| C | | 84,5 | -0,6 | 7,5 | 40,3 |
| D | | 84,5 | -0,8 | 7,4 | 41,0 |
| E | | 84,6 | -0,4 | 7,5 | 40,8 |
| A | 90 °C | 85,7 | -4,4 | 7,4 | 40,9 |
| B | | 85,9 | -4,5 | 7,4 | 41,4 |
| C | | 86,6 | -3,4 | 7,2 | 41,8 |
| D | | 86,8 | -3,4 | 7,4 | 40,9 |
| E | | 86,8 | -3,2 | 7,4 | 41,0 |

| | | | | | |
|---|---|---|---|---|---|
| * moyenne sur l'enregistrement de l'essai (1 valeur toutes les secondes) ** calculé sur l'enregistrement de l'essai (température de la filière- température de la préparation en sortie de conditionneur) *** calculé à partir du poids de 20 cm de granulés **** mesuré sur un prélèvement de 30 secondes | | | | | |

Les consignes de régulation de la presse sont les suivantes:
Débit : environ 41 kg/h,
Températures de traitement en sortie conditionneur : 80, 85 et 90°C,
Pression de vapeur : 1,6.10⁵ Pa,
Réglage de la hauteur de coupe des couteaux : 10 mm.

### 2. Protocole expérimental de mesure de l'activité enzymatique

### 2.1. β-glucanase par la méthode au DNS

L'essai se base sur l'hydrolyse enzymatique de β-glucane de l'orge, un β-1,3(4)-glucane. Les produits de la réaction sont déterminés par colorimétrie en mesurant l'augmentation des groupes réducteurs à l'aide d'acide 3,5-dinitrosalicylique (DNS). La concentration en sucre réducteur disponible après hydrolyse enzymatique est déterminée à l'aide d'une courbe de glucose étalon dont l'absorbance est mesurée à 540 nm. L'activité enzymatique calculée est ensuite exprimée en équivalents glucose.

Une solution contenant 1 ml d'une solution de β-glucane à 1% (m/V) dans un tampon acétate de sodium 0,1 M à pH 5,0 et 1 ml de solution de l'enzyme à la dilution appropriée est incubée à 50°C pendant 10 minutes. La réaction enzymatique est stoppée par l'ajout de 2 ml d'une solution de DNS (1% (m/V) d'acide 3,5-dinitrosalicylique, 1,6% (m/V) de NaOH, 30% (m/V) de tartrate de potassium et sodium (+) dans de l'eau distillée). La solution est homogénéisée puis placée dans un bain-marie bouillant à 95°C minimum et ensuite refroidie dans un bain à la température ambiante (pendant 5 minutes). Dix millilitres d'eau ultrapure sont ajoutés à la solution et l'absorbance est mesurée à 540 nm dans une cellule en verre ayant une longueur de trajet optique de 1 cm.

L'absorbance est corrigée avec celle obtenue pour une solution de référence à laquelle le DNS est ajouté avant la solution enzymatique.

Les résultats sont convertis en µmoles de sucre réducteur par comparaison avec une gamme de solutions étalons allant de 0,00 à 0,04% (m/V) de glucose, traitées au DNS comme dans le cas des essais.

Une unité d'activité d'endo-1,3(4)-β-glucanase est définie comme la quantité d'enzyme qui produit 1 µmole d'équivalent glucose par minute et par gramme de produit, dans les conditions de l'essai (pH 5,0 et 50°C).

### 2.2. Xylanase par la méthode au DNS

L'essai se base sur l'hydrolyse enzymatique de xylane de bouleau, un polymère de xylose contenant des ponts β-D-1,4. Les produits de la réaction sont déterminés par colorimétrie en mesurant l'augmentation des groupes réducteurs à l'aide d'acide 3,5-dinitrosalicylique. La concentration en sucre réducteur disponible après hydrolyse enzymatique est déterminée à l'aide d'une courbe de xylose étalon dont l'absorbance est mesurée à 540 nm. L'activité enzymatique calculée est ensuite exprimée en équivalents xylose.

Une solution contenant 1 ml d'une solution de xylane de bouleau à 1% (m/V) dans un tampon acétate de sodium 0,1 M à pH 5,0 et 1 ml de solution de l'enzyme à la dilution appropriée est incubée à 50°C pendant 10 minutes. La réaction enzymatique est stoppée par l'ajout de 2 ml d'une solution de DNS (1% (m/V) d'acide 3,5-dinitrosalicylique, 1,6% (m/V) de NaOH, 30% (m/V) de tartrate de potassium et sodium (+) dans de l'eau distillée). La solution est homogénéisée puis placée dans un bain-marie bouillant à 95°C minimum et ensuite refroidie dans un bain à la température ambiante (pendant 5 minutes). Dix millilitres d'eau ultrapure sont ajoutés à la solution et l'absorbance est mesurée à 540 nm dans une cellule en verre ayant une longueur de trajet optique de 1 cm.

L'absorbance est corrigée avec celle obtenue pour une solution de référence à laquelle le DNS est ajouté avant la solution enzymatique.

Les résultats sont convertis en µmoles de sucre réducteur par comparaison avec une gamme de solutions étalons allant de 0,00 à 0,04% (m/V) de xylose, traitées au DNS comme dans le cas des essais.

Une unité d'activité d'endo-1,4-β-xylanase est définie comme la quantité d'enzyme qui produit 1 µmole d'équivalent xylose par minute et par gramme de produit, dans les conditions de l'essai (pH 5,0 et 50°C).

### 2.3. Xylanase par la méthode viscosimétrie

Cette méthode est spécifique à la détermination de l'activité endo-1,4-β-xylanase dans les aliments. L'endo-1,4-β-xylanase hydrolyse les ponts xylosidiques du xylane. L'essai est basé sur l'hydrolyse enzymatique des ponts xylosiques d'une solution d'arabinose de blé, polysaccharide de β-1,4-xylane substitué avec de l'arabinose. L'activité enzymatique est proportionnelle à la réduction de viscosité d'une solution d'arabinoxylane de blé en présence de l'enzyme à doser.

Une unité d'activité d'endo-1,4-β-xylanase est définie comme la quantité d'enzyme qui hydrolysera le substrat, réduisant la viscosité de la solution, pour donner un changement de la fluidité relative de 1 unité sans dimensions par minute dans les conditions de l'analyse : pH 5,5 et 30 °C.

### 3. Résultats

**Tableau 5 : Contrôle des activités enzymatiques des compositions pulvérulentes (avant passage en presse)**

| | Concentré Bruxel SD | Produit standard | Compositions pulvérulentes de l'invention | | |
|---|---|---|---|---|---|
| Présent dans la préparation | - | A et C | B | D | E |
| Activité enzymatique (unités/g) | | | | | |
| Xylanase DNS | 6507 | 3810 | 4027 | 5344 | 5304 |
| Xylanase visco. | 39065 | 31053 | 30293 | 32273 | 31153 |
| β-glutanase DNS | 7626 | 4676 | 5265 | 6300 | 6081 |

Ces résultats montrent bien que les principales activités enzymatiques sont préservées dans les différentes compositions pulvérulentes, en référence au concentré enzymatique initial (concentré Bruxel SD).

**Tableau 6 : Taux de récupération en activité enzymatique* mesurée sur les aliments après passage en presse à différentes températures**

| **% de rétention de l'activité enzymatique dans le produit final après passage en presse** | **Température de traitement presse** | | | **Taux de CMC en % de l'extrait sec** |
|---|---|---|---|---|
| | **80 °C** | **85 °C** | **90 °C** | |
| Test 1 | | | | |
| Produit témoin sans protection (A) | 76% | 68% | 54% | **0%** |
| Produit avec protection (B) | 100% | 85% | 82% | **55%** |

| Test 2 | | | | |
|---|---|---|---|---|
| Produit témoin sans protection (C) | 86% | 57% | 40% | **0%** |
| Produit avec protection (D) | 99% | 90% | 81% | **35%** |
| Produit avec protection (E) | 96% | 86% | 83% | **35%** |

| | | | | |
|---|---|---|---|---|
| * activité xylanase mesurée par viscométrie | | | | |

Les résultats figurant dans le tableau 6 ci-dessus démontrent la stabilité de la substance sensible conférée par la composition pulvérulente selon l'invention.

A 80 °C, l'enzyme conserve 100% de son activité, alors que l'enzyme non protégée a perdu 1/4 de son activité (teste 1). A des températures supérieures à 85 °C, l'enzyme conserve plus de 80% de son activité.

On note qu'une protection équivalente est obtenue avec le test 1 ou 2, qui correspondent à des compositions pulvérulentes contenant 55% et 35% de CMC, respectivement. La plus grande compaction et densification des particules obtenues avec 35% de CMC permettent ainsi de conserver la stabilité de l'enzyme même avec une quantité moindre de CMC.

On pourrait envisager de diminuer encore le pourcentage de CMC utilisée, et notamment de descendre à 20% en CMC, en gardant le même niveau de protection des enzymes.

## Revendications

1. Utilisation d'au moins un composé visqueux, dont la viscosité est supérieure à 5 Pa.s¹, pour empêcher substantiellement la dégradation d'une substance sensible contenue dans une composition pulvérulente stable, ladite substance sensible subissant moins de 20% de dégradation lorsque ladite composition pulvérulente stable est soumise à des températures de 60°C à 100°C et/ou à des pressions de 2.10⁶ à 10⁷ Pa et/ou à une humidité relative de 60% à 100% pendant l'étape de granulation de ladite composition, ladite composition pulvérulente contenant des particules comprenant :
- un coeur contenant ladite substance sensible, au moins un composé visqueux en tant qu'agent d'imprégnation de la substance sensible, et au moins un support de la substance sensible, et
- un enrobage du coeur, lequel enrobage par au moins un composé visqueux en tant qu'agent d'engluage, lesdits composés visqueux étant choisis parmi la carboxyméthylcellulose (CMC), et
la viscosité étant mesurée à l'aide d'un viscosimètre BROOKFIELD modèle LVDV-E équipé de système à cylindre coaxial, à l'aide des mobiles n° 18 ou 31 selon les viscosités, dans une chambre de mesure à température contrôlée à l'aide d'un bain thermostaté, ces mesures étant effectuées à 20°C pour des solutions aqueuses à 10% d'extrait sec du composé visqueux, à différentes vitesses de rotation des mobiles de 10 à 100 rpm, les valeurs de viscosité retenues étant les valeurs obtenues aux vitesses de rotation les plus élevées en intégrant les valeurs limites du couple torsion (80% maximum) de l'appareil.

2. Utilisation selon la revendication 1, dans laquelle la substance sensible représente de 5 à 40%, préférentiellement de 10 à 30% d'extrait sec de la composition pulvérulente, le support représente de 10 à 60%, préférentiellement de 20 à 50% d'extrait sec de la composition pulvérulente, l'agent d'imprégnation représente de 5 à 40%, préférentiellement de 10 à 30% d'extrait sec de la composition pulvérulente et l'agent d'engluage représente de 5 à 30%, préférentiellement de 10 à 20% d'extrait sec de la composition pulvérulente.

3. Utilisation selon la revendication 1, dans laquelle la substance sensible est constituée par un mélange d'enzymes constitué principalement de β-glutanase, de xylanase et de cellulase, le support est constitué par de la farine de blé, l'agent d'imprégnation et l'agent d'engluage sont constitués par la carboxyméthylcellulose (CMC).

4. Composition pulvérulente stable contenant des particules comprenant :
- un coeur contenant au moins une substance sensible subissant moins de 20% de dégradation lorsque ladite composition pulvérulente stable est soumise à des températures de 60°C à 100°C et/ou à des pressions de 2.10⁶ à 10⁷ Pa et/ou à une humidité relative de 60% à 100% pendant l'étape de granulation de ladite composition, et au moins un composé visqueux dont la viscosité est supérieure à 5 Pa.s⁻¹ en tant qu'agent d'imprégnation de la substance sensible, et au moins un support de la substance sensible, et
- un enrobage du coeur, lequel enrobage par au moins un composé visqueux dont la viscosité est supérieure à 5 Pa.s⁻¹ en tant qu'agent d'engluage,
dans laquelle la substance sensible n'est pas une enzyme destinée à l'alimentation d'animaux non humains, lesdits composés visqueux étant choisis parmi la carboxyméthylcellulose (CMC), et la viscosité étant mesurée selon la méthode définie dans la revendication 1.

5. Composition pulvérulente stable selon la revendication 4, dans laquelle ladite substance sensible est choisie parmi les protéines, les vitamines, les bactéries, les levures, les anti-oxydants, les caroténoïdes, les huiles essentielles ou des substances pharmacologiquement actives telles que les antibiotiques.

6. Composition pulvérulente stable selon la revendication 4, dans laquelle le support est choisi parmi l'amidon, les farines, notamment de blé, de maïs, de manioc ou de riz, le talc, la pulpe de betterave, la maltodextrine, les sels tels que le carbonate de calcium, les drèches de maïs.

7. Composition pulvérulente stable selon la revendication 4, dans laquelle la substance sensible représente de 5 à 40%, préférentiellement de 10 à 30% d'extrait sec de la composition pulvérulente, le support représente de 10 à 60%, préférentiellement de 20 à 50% d'extrait sec de la composition pulvérulente, l'agent d'imprégnation représente de 5 à 40%, préférentiellement de 10 à 30% d'extrait sec de la composition pulvérulente et l'agent d'engluage représente de 5 à 30%, préférentiellement de 10 à 20% d'extrait sec de la composition pulvérulente.

8. Procédé de préparation d'une composition pulvérulente stable contenant des particules comprenant :
- un coeur contenant au moins une substance sensible subissant moins de 20% de dégradation lorsque ladite composition pulvérulente stable est soumise à des températures de 60°C à 100°C et/ou à des pressions de 2.10⁶ à 10⁷ Pa et/ou à une humidité relative de 60% à 100% pendant l'étape de granulation de ladite composition, au moins un composé visqueux dont la viscosité est supérieure à 5 Pa.s⁻¹ en tant qu'agent d'imprégnation de la substance sensible et au moins un support de la substance sensible, et
- un enrobage du coeur, lequel enrobage par au moins un composé visqueux dont la viscosité est supérieure à 5 Pa.s⁻¹ en tant qu'agent d'engluage, lesdits composés visqueux étant choisis parmi la carboxyméthylcellulose (CMC), et la viscosité étant mesurée selon la méthode définie dans la revendication 1,
ledit procédé comprenant :
une étape de microgranulation d'un mélange de ladite substance sensible, dudit support et dudit agent d'imprégnation pour obtenir le coeur, et
une étape d'enrobage dudit coeur par ledit agent d'engluage.

9. Procédé selon la revendication 8, comprenant une étape de microgranulation d'un mélange de ladite substance sensible et dudit support co-séchés et de la totalité de l'agent d'imprégnation.

10. Procédé selon la revendication 8, comprenant une étape de microgranulation d'un mélange de ladite substance sensible, dudit support et de la totalité de l'agent d'imprégnation, co-séchés.

11. Procédé selon la revendication 8, comprenant une étape de microgranulation d'un mélange de ladite substance sensible, dudit support et d'une partie de l'agent d'imprégnation, co-séchés et du reste de l'agent d'imprégnation.

12. Procédé selon la revendication 8, comprenant une étape de co-séchage d'un mélange contenant la substance sensible, notamment sous forme liquide, le support de la substance sensible, notamment sous forme sèche, et éventuellement tout ou partie de l'agent d'imprégnation, le co-séchage étant effectué à une température d'air de sortie inférieure à 60 °C et à une température de poudre inférieure à 45 °C.

13. Procédé selon l'une des revendications 8 à 12, comprenant:
a. une étape de co-séchage d'un mélange contenant la substance sensible, notamment sous forme liquide, et au moins un support de la substance sensible, notamment sous forme sèche, le co-séchage étant effectué à une température d'air de sortie inférieure à 60 °C et à une température de poudre inférieure à 45 °C pour obtenir une poudre homogène intermédiaire, et
b. une étape de microgranulation de la poudre homogène intermédiaire avec l'agent d'imprégnation, de préférence sous forme solide, pour obtenir une poudre microgranulée densifiée constituée de particules non enrobées

14. Procédé de préparation selon l'une des revendications 8 à 13, comprenant :
a. une étape de co-séchage d'un mélange contenant la substance sensible, notamment sous forme liquide, et au moins un support de la substance sensible, notamment sous forme sèche, le co-séchage étant effectué à une température d'air de sortie inférieure à 60 °C et à une température de poudre inférieure à 45 °C, pour obtenir une poudre homogène intermédiaire,
b. une étape de microgranulation de la poudre homogène intermédiaire avec l'agent d'imprégnation, de préférence sous forme solide, pour obtenir une poudre microgranulée densifiée constituée de particules non enrobées correspondant au susdit coeur,
c. une étape d'enrobage des particules non enrobées de la poudre microgranulée densifiée avec l'agent d'engluage, de préférence sous forme liquide, pour obtenir une poudre enrobée.

15. Procédé de préparation selon l'une des revendications 8 à 14, comprenant :
a. une étape de co-séchage d'un mélange contenant la substance sensible, notamment sous forme liquide, et au moins un support de la substance sensible, notamment sous forme sèche, le co-séchage étant effectué à une température d'air de sortie inférieure à 60 °C et à une température de poudre inférieure à 45 °C, pour obtenir une poudre homogène intermédiaire,
b. une étape de microgranulation de la poudre homogène intermédiaire avec l'agent d'imprégnation, de préférence sous forme solide, pour obtenir une poudre microgranulée densifiée constituée de particules non enrobées correspondant au susdit coeur,
c. éventuellement une étape de séchage à basse température de la poudre microgranulée densifiée à une température inférieure à 45°C, pour obtenir une poudre microgranulée densifiée séchée,
d. éventuellement une étape de tamisage pour obtenir une poudre microgranulée densifiée tamisée,
e. une étape d'enrobage des particules non enrobées de la poudre microgranulée densifiée éventuellement séchée et éventuellement tamisée, avec l'agent d'engluage, de préférence sous forme liquide, pour obtenir une poudre enrobée,
f. une étape de séchage de la poudre enrobée pour obtenir une composition pulvérulente stable.

16. Composition pharmaceutique contenant une composition pulvérulente stable contenant des particules comprenant
- un coeur contenant, comme substance active, au moins une substance sensible subissant moins de 20% de dégradation lorsque ladite composition pulvérulente stable est soumise à des températures de 60°C à 100°C et/ou à des pressions de 2.10⁶ à 10⁷ Pa et/ou à une humidité relative de 60% à 100% pendant l'étape de granulation de ladite composition, et au moins un composé visqueux dont la viscosité est supérieure à 5 Pa.s⁻¹ en tant qu'agent d'imprégnation de la substance sensible, et au moins un support de la substance sensible, et
- un enrobage du coeur, lequel enrobage par au moins un composé visqueux dont la viscosité est supérieure à 5 Pa.s⁻¹ en tant qu'agent d'engluage, dans laquelle la substance sensible possède au moins 80%, notamment au moins 90% de son activité initiale, et lesdits composés visqueux étant choisis parmi la carboxyméthylcellulose (CMC), et la viscosité étant mesurée selon la méthode définie dans la revendication 1,
en association avec un véhicule pharmaceutiquement acceptable.

17. Composition alimentaire comprenant une composition pulvérulente stable selon l'une des revendications 4 à 7, dans laquelle la substance sensible possède au moins 80%, notamment au moins 90%, de son activité initiale et n'est pas une enzyme destinée à l'alimentation d'animaux non humains, et au moins un produit alimentaire.

18. Composition alimentaire selon la revendication 17, **caractérisée en ce qu'**elle se présente sous la forme de pellets ou de granulés.

## Patentansprüche

1. Verwendung mindestens einer viskosen Verbindung, deren Viskosität größer als 5 Pa.s¹ ist, um den Abbau einer empfindlichen Substanz, die in einer stabilen Pulverzusammensetzung enthalten ist, im Wesentlichen zu verhindern, wobei die empfindliche Substanz zu weniger als 20 % abgebaut wird, wenn die stabile Pulverzusammensetzung Temperaturen von 60 °C bis 100 °C und/oder Drücken von 2.10⁶ bis 10⁷ Pa und/oder einer relativen Feuchtigkeit von 60 % bis 100 % während des Schritts zur Granulierung der Zusammensetzung ausgesetzt wird, wobei die Pulverzusammensetzung Teilchen enthält, umfassend:
- einen Kern, der die empfindliche Substanz enthält, mindestens eine viskose Verbindung als Imprägniermittel der empfindlichen Substanz und mindestens einen Träger der empfindlichen Substanz, und
- eine Beschichtung des Kerns, und zwar eine Beschichtung mit mindestens einer viskosen Verbindung als Haftmittel,
wobei die viskosen Verbindungen ausgewählt sind aus Carboxymethylcellulose (CMC) und
die Viskosität unter Verwendung eines BROOKFIELD-Viskosimeters, Modell LVDE-E, ausgestattet mit einem Koaxialzylindersystem, unter Verwendung der Spindeln Nr. 18 oder 31 entsprechend den Viskositäten in einer unter Verwendung eines Heizbads temperaturgeregelten Messkammer gemessen wird, wobei diese Messungen für wässrige Lösungen mit 10 % Trockenextraktanteil der viskosen Verbindung bei 20 °C mit unterschiedlichen Drehgeschwindigkeiten der Spindeln von 10 bis 100 U/min durchgeführt werden, wobei die berücksichtigten Viskositätswerte die Werte sind, die bei den höchsten Drehgeschwindigkeiten unter Einbeziehung der Grenzwerte des Drehmoments (maximal 80 %) der Vorrichtung erhalten werden.

2. Verwendung nach Anspruch 1, wobei die empfindliche Substanz 5 bis 40 %, vorzugsweise 10 bis 30 % Trockenextraktanteil der Pulverzusammensetzung ausmacht, der Träger 10 bis 60 %, vorzugsweise 20 bis 50 % Trockenextraktanteil der Pulverzusammensetzung ausmacht, das Imprägniermittel 5 bis 40 %, vorzugsweise 10 bis 30 % Trockenextraktanteil der Pulverzusammensetzung ausmacht und das Haftmittel 5 bis 30 %, vorzugsweise 10 bis 20 % Trockenextraktanteil der Pulverzusammensetzung ausmacht.

3. Verwendung nach Anspruch 1, wobei die empfindliche Substanz aus einem Enzymgemisch besteht, das hauptsächlich aus β-Glutanase, Xylanase und Cellulase besteht, wobei der Träger aus Weizenmehl besteht, das Imprägniermittel und das Haftmittel aus Carboxymethylcellulose (CMC) bestehen.

4. Stabile Pulverzusammensetzung, die Teilchen enthält, umfassend:
- einen Kern, der mindestens eine empfindliche Substanz enthält, die zu weniger als 20 % abgebaut wird, wenn die stabile Pulverzusammensetzung Temperaturen von 60 °C bis 100 °C und/oder Drücken von 2.10⁶ bis 10⁷ Pa und/oder einer relativen Feuchtigkeit von 60 % bis 100 % während des Schritts zur Granulierung der Zusammensetzung ausgesetzt wird, und mindestens eine viskose Verbindung, deren Viskosität größer 5 Pa.s-¹ ist, als Imprägniermittel der empfindlichen Substanz und mindestens einen Träger der empfindlichen Substanz, und
- eine Beschichtung des Kerns, und zwar eine Beschichtung durch mindestens eine viskose Verbindung, deren Viskosität größer als 5 Pa.s-¹ ist, als Haftmittel,
wobei die empfindliche Substanz kein Enzym ist, das zur Ernährung von nichthumanen Tieren bestimmt ist,
wobei die viskosen Verbindungen ausgewählt sind aus Carboxymethylcellulose (CMC) und die Viskosität gemäß der in Anspruch 1 definierten Methode gemessen wird.

5. Stabile Pulverzusammensetzung nach Anspruch 4, wobei die empfindliche Substanz ausgewählt ist aus Proteinen, Vitaminen, Bakterien, Hefen, Antioxidationsmitteln, Carotinoiden, ätherischen Ölen oder pharmakologisch aktiven Substanzen, wie etwa Antibiotika.

6. Stabile Pulverzusammensetzung nach Anspruch 4, wobei der Träger ausgewählt ist aus Stärke, Mehlen, insbesondere Weizen-, Mais-, Maniok- oder Reismehlen, Talk, Rübenpulpe, Maltodextrin, Salzen, wie etwa Calciumcarbonat, Maisschlempe.

7. Stabile Pulverzusammensetzung nach Anspruch 4, wobei die empfindliche Substanz 5 bis 40 %, vorzugsweise 10 bis 30 % Trockenextraktanteil der Pulverzusammensetzung ausmacht, der Träger 10 bis 60 %, vorzugsweise 20 bis 50 % Trockenextraktanteil der Pulverzusammensetzung ausmacht, das Imprägniermittel 5 bis 40 %, vorzugsweise 10 bis 30 % Trockenextraktanteil der Pulverzusammensetzung ausmacht und das Haftmittel 5 bis 30 %, vorzugsweise 10 bis 20 % Trockenextraktanteil der Pulverzusammensetzung ausmacht.

8. Verfahren zur Herstellung einer stabilen Pulverzusammensetzung, die Teilchen enthält, umfassend:
- einen Kern, der mindestens eine empfindliche Substanz enthält, die zu weniger als 20 % abgebaut wird, wenn die stabile Pulverzusammensetzung Temperaturen von 60 °C bis 100 °C und/oder Drücken von 2.10⁶ bis 10⁷ Pa und/oder einer relativen Feuchtigkeit von 60 % bis 100 % während des Schritts zur Granulierung der Zusammensetzung ausgesetzt wird, mindestens eine viskose Verbindung, deren Viskosität größer als 5 Pa.s-¹ ist, als Imprägniermittel der empfindlichen Substanz und mindestens einen Träger der empfindlichen Substanz, und
- eine Beschichtung des Kerns, und zwar eine Beschichtung durch mindestens eine viskose Verbindung, deren Viskosität größer als 5 Pa.s-¹ ist, als Haftmittel,
wobei die viskosen Verbindungen ausgewählt sind aus Carboxymethylcellulose (CMC) und die Viskosität gemäß der in Anspruch 1 definierten Methode gemessen wird,
wobei das Verfahren umfasst:
einen Schritt zur Mikrogranulierung eines Gemischs der empfindlichen Substanz, des Trägers und des Imprägniermittels, um den Kern zu erhalten, und
einen Schritt zum Beschichten des Kerns mit dem Haftmittel.

9. Verfahren nach Anspruch 8, umfassend einen Schritt zum Mikrogranulieren eines Gemischs aus der empfindlichen Substanz und dem Träger, die zusammen getrocknet werden, und dem gesamten Imprägniermittel.

10. Verfahren nach Anspruch 8, umfassend einen Schritt zum Mikrogranulieren eines Gemischs aus der empfindlichen Substanz und dem Träger, die zusammen getrocknet werden, und dem gesamten Imprägniermittel.

11. Verfahren nach Anspruch 8, umfassend einen Schritt zum Mikrogranulieren eines Gemischs aus der empfindlichen Substanz, dem Träger und einem Teil des Imprägniermittels, die zusammen getrocknet werden, und dem Rest des Imprägniermittels.

12. Verfahren nach Anspruch 8, umfassend einen Schritt zum gemeinsamen Trocknen eines Gemischs, das die empfindliche Substanz, insbesondere in flüssiger Form, den Träger der empfindlichen Substanz, insbesondere in trockener Form, und gegebenenfalls das gesamte oder ein Teil des Imprägniermittels enthält, wobei das gemeinsame Trocknen bei einer Abluftlufttemperatur kleiner als 60 °C und einer Pulvertemperatur kleiner als 45 °C durchgeführt wird.

13. Verfahren nach einem der Ansprüche 8 bis 12, umfassend:
a. einen Schritt zum gemeinsamen Trocknen eines Gemischs, das die empfindliche Substanz, insbesondere in flüssiger Form, und mindestens einen Träger der empfindlichen Substanz, insbesondere in trockener Form, enthält, wobei das gemeinsame Trocknen bei einer Ablufttemperatur kleiner als 60 °C und einer Pulvertemperatur kleiner als 45 °C durchgeführt wird, um ein homogenes Intermediärpulver zu erhalten, und
b. einen Schritt zum Mikrogranulieren des homogenen Intermediärpulvers mit dem Imprägniermittel, vorzugsweise in fester Form, um ein verdichtetes mikrogranuliertes Pulver zu erhalten, das aus unbeschichteten Teilchen besteht.

14. Herstellungsverfahren nach einem der Ansprüche 8 bis 13, umfassend:
a. einen Schritt zum gemeinsamen Trocknen eines Gemischs, das die empfindliche Substanz, insbesondere in flüssiger Form, und mindestens einen Träger der empfindlichen Substanz, insbesondere in trockener Form, enthält, wobei das gemeinsame Trocknen bei einer Ablufttemperatur kleiner als 60 °C und einer Pulvertemperatur kleiner als 45 °C durchgeführt wird, um ein homogenes Intermediärpulver zu erhalten,
b. einen Schritt zum Mikrogranulieren des homogenen Intermediärpulvers mit dem Imprägniermittel, vorzugsweise in fester Form, um ein verdichtetes mikrogranuliertes Pulver zu erhalten, das aus unbeschichteten Teilchen besteht, die dem vorgenannten Kern entsprechen,
c. einem Schritt zum Beschichten der unbeschichteten Teilchen des verdichteten mikrogranulierten Pulvers mit dem Haftmittel, vorzugsweise in flüssiger Form, um ein beschichtetes Pulver zu erhalten.

15. Herstellungsverfahren nach einem der Ansprüche 8 bis 14, umfassend:
a. einen Schritt zum gemeinsamen Trocknen eines Gemischs, das die empfindliche Substanz, insbesondere in flüssiger Form, und mindestens einen Träger der empfindlichen Substanz, insbesondere in trockener Form, enthält, wobei das gemeinsame Trocknen bei einer Ablufttemperatur kleiner als 60 °C und einer Pulvertemperatur kleiner als 45 °C durchgeführt wird, um ein homogenes Intermediärpulver zu erhalten,
b. einen Schritt zum Mikrogranulieren des homogenen Intermediärpulvers mit dem Imprägniermittel, vorzugsweise in fester Form, um ein verdichtetes mikrogranuliertes Pulver zu erhalten, das aus unbeschichteten Teilchen besteht, die dem vorgenannten Kern entsprechen,
c. gegebenenfalls einen Schritt zum Trocknen bei niedriger Temperatur des verdichteten mikrogranulierten Pulvers bei einer Temperatur kleiner als 45 °C, um ein getrocknetes, verdichtetes, mikrogranuliertes Pulver zu erhalten,
d. gegebenenfalls einen Schritt zum Sieben, um zu ein gesiebtes, verdichtetes, mikrogranuliertes Pulver zu erhalten,
e. einem Schritt zum Beschichten der unbeschichteten Teilchen des gegebenenfalls getrockneten und gegebenenfalls gesiebten, verdichteten mikrogranulierten Pulvers mit dem Haftmittel, vorzugsweise in flüssiger Form, um ein beschichtetes Pulver zu erhalten.
f. einen Schritt zum Trocknen des beschichteten Pulvers, um eine stabile Pulverzusammensetzung zu erhalten.

16. Pharmazeutische Zusammensetzung, enthaltend eine stabile Pulverzusammensetzung, die Teilchen enthält, umfassend:
- einen Kern, der als Wirkstoff mindestens eine empfindliche Substanz enthält, die zu weniger als 20 % abgebaut wird, wenn die stabile Pulverzusammensetzung Temperaturen von 60 °C bis 100 °C und/oder Drücken von 2.10⁶ bis 10⁷ Paund/oder einer relativen Feuchtigkeit von 60 % bis 100 % während des Schritts zur Granulierung der Zusammensetzung ausgesetzt wird, und mindestens eine viskose Verbindung, deren Viskosität größer als 5 Pa.s-¹ ist, als Imprägniermittel der empfindlichen Substanz und mindestens einen Träger der empfindlichen Substanz, und
- eine Beschichtung des Kerns, und zwar eine Beschichtung durch mindestens eine viskose Verbindung, deren Viskosität größer als 5 Pa.s-¹ ist, als Haftmittel,
wobei die empfindliche Substanz mindestens 80 %, insbesondere mindestens 90 % ihrer ursprünglichen Aktivität aufweist, und
wobei die viskosen Verbindungen ausgewählt sind aus Carboxymethylcellulose (CMC) und die Viskosität gemäß der in Anspruch 1 definierten Methode gemessen wird,
in Verbindung mit einem pharmazeutisch verträglichen Vehikel.

17. Nahrungsmittelzusammensetzung, umfassend eine stabile Pulverzusammensetzung nach einem der Ansprüche 4 bis 7, wobei die empfindliche Substanz mindestens 80 %, insbesondere mindestens 90 % ihrer Anfangsaktivität aufweist und kein Enzym ist, das für die Ernährung nichthumaner Tiere bestimmt ist, und mindestens ein Nahrungsmittelprodukt.

18. Nahrungsmittelzusammensetzung nach Anspruch 17, **dadurch gekennzeichnet, dass** sie in Form von Pellets oder Granulat vorliegt.

## Claims

1. Use of at least one viscous compound, the viscosity of which is greater than 5 Pa.s⁻¹, to substantially prevent the degradation of a sensitive substance comprised in a stable powder composition, said sensitive substance undergoing less than 20% degradation when said powder composition is subjected to temperatures of 60°C to 100°C and/or pressures of 2x10⁶ to 10⁷ Pa and/or a relative humidity of 60% to 100%, during the granulation step of said composition, said powder composition containing particles comprising:
- a core containing said sensitive substance, at least one viscous compound as impregnating agent for the sensitive substance, and at least a support of the sensitive substance, and
- a coating for the core, which contains at least one viscous compound as sticking agent,
said viscous compounds being chosen from carboxymethyl cellulose (CMC), and the viscosity being measured using a BROOKFIELD viscometer, model LVDV-E fitted with a coaxial cylinder system, using spindles no. 18 or 31 depending on the viscosities, in a measurement chamber with a temperature controlled using a thermostated bath, these measurements being carried out at 20°C for aqueous solutions with 10% dry extract of the viscous compound, at different rotational speeds of the spindle of from 10 to 100 rpm, the retained viscosity values being the values obtained at the highest rotational speeds, integrating the limit values of the torsion torque (maximum 80%) of the device.

2. Use according to claim 1, in which the sensitive substance represents 5 to 40%, preferably 10 to 30% of the dry extract of the powder composition, the support represents 10 to 60%, preferably 20 to 50% of the dry extract of the powder composition, the impregnating agent represents 5 to 40%, preferably 10 to 30% of the dry extract of the powder composition and the sticking agent represents 5 to 30%, preferably 10 to 20% of the dry extract of the powder composition.

3. Use according to claim 1, in which the sensitive substance is constituted by a mixture of enzymes constituted principally by β-glucanase, xylanase and cellulase, the support is constituted by wheat flour, the impregnating agent and the sticking agent are constituted by carboxymethyl cellulose (CMC).

4. Stable powder composition containing particles comprising:
- a core containing at least one sensitive substance undergoing less than 20% degradation when said powder composition is subjected to temperatures of 60°C to 100°C and/or pressures of 2x10⁶ to 10⁷ Pa and/or a relative humidity of 60% to 100% during the granulation step of said composition, and at least one viscous compound the viscosity of which is greater than 5 Pa.s⁻¹ as impregnating agent for the sensitive substance, and at least a support of the sensitive substance, and
- a coating for the core, which contains at least one viscous compound the viscosity of which is greater than 5 Pa.s⁻¹ as sticking agent,
in which the sensitive substance is not an enzyme intended for non-human animals,
said viscous compounds being chosen from carboxymethyl cellulose (CMC), and the viscosity being measured according to the method defined in claim 1.

5. Stable powder composition according to claim 4, in which said sensitive substance is chosen from proteins, vitamins, bacteria, yeasts, antioxidants, carotenoids, essential oils or pharmacologically active substances such as antibiotics.

6. Stable powder composition according to claim 4, in which the support is chosen from starch, flours, in particular wheat, corn, manioc or rice flour, talc, beet pulp, maltodextrin, salts such as calcium carbonate, corn distiller's grain.

7. Stable powder composition according to claim 4, in which the sensitive substance represents 5 to 40%, preferably 10 to 30% of the dry extract of the powder composition, the support represents 10 to 60%, preferably 20 to 50% of the dry extract of the powder composition, the impregnating agent represents 5 to 40%, preferably 10 to 30% of the dry extract of the powder composition and the sticking agent represents 5 to 30%, preferably 10 to 20% of the dry extract of the powder composition.

8. Process for preparing a stable powder composition containing particles comprising:
- a core containing at least one sensitive substance undergoing less than 20% degradation when said powder composition is subjected to temperatures of 60°C to 100°C and/or pressures of 2x10⁶ to 10⁷ Pa and/or a relative humidity of 60% to 100% during the granulation step of said composition, and at least one viscous compound the viscosity of which is greater than 5 Pa.s⁻¹ as impregnating agent for the sensitive substance, and at least a support of the sensitive substance, and
- a coating for the core, which contains at least one viscous compound the viscosity of which is greater than 5 Pa.s⁻¹ as sticking agent,
said viscous compounds being chosen from carboxymethyl cellulose (CMC), and the viscosity being measured according to the method defined in claim 1,
said method comprising:
a microgranulation step for a mixture of said sensitive substance, said support and said impregnating agent in order to obtain the core, and
a step of coating said core with said sticking agent.

9. Process according to claim 8, comprising a microgranulation step of a mixture of said sensitive substance and said support, which have been co-dried, and all of the impregnating agent.

10. Process according to claim 8, comprising a microgranulation step for a mixture of said sensitive substance, said support and all of the impregnating agent, which have been co-dried.

11. Process according to claim 8, comprising a microgranulation step for a mixture of said sensitive substance, said support and some of the impregnating agent, which have been co-dried, and the remainder of the impregnating agent.

12. Process according to claim 8, comprising a co-drying step of a mixture containing the sensitive substance, in particular in liquid form, the support of the sensitive substance, in particular in dry form, and optionally all or a part of the impregnating agent, the co-drying being carried out at an output air temperature of less than 60°C and at a powder temperature of less than 45°C.

13. Process according to anyone of claims 8 to 12, comprising:
**a.** a co-drying step for a mixture containing the sensitive substance, in particular in liquid form, and at least one support of the sensitive substance, in particular in dry form, the co-drying being carried out at an output air temperature of less than 60°C and at a powder temperature of less than 45°C, in order to obtain an intermediate homogeneous powder, and
**b.** a microgranulation step of the intermediate homogeneous powder with the impregnating agent, preferably in solid form, in order to obtain a densified microgranulated powder constituted by uncoated particles.

14. Process of preparation according to anyone of claims 8 to 13, comprising:
**a.** a co-drying step for a mixture containing the sensitive substance, in particular in liquid form, and at least one support of the sensitive substance, in particular in dry form, the co-drying being carried out at an output air temperature of less than 60°C and at a powder temperature of less than 45°C, in order to obtain an intermediate homogeneous powder,
**b.** a microgranulation step for the intermediate homogeneous powder with the impregnating agent, preferably in solid form, in order to obtain a densified microgranulated powder constituted by uncoated particles corresponding to the abovementioned core,
**c.** a step of coating the uncoated particles of the densified microgranulated powder with the sticking agent, preferably in liquid form, in order to obtain a coated powder.

15. Process of preparation according to anyone of claims 8 to 14, comprising:
**a.** a co-drying step for a mixture containing the sensitive substance, in particular in liquid form, and at least one support of the sensitive substance, in particular in dry form, the co-drying being carried out at an output air temperature of less than 60°C and at a powder temperature of less than 45°C, in order to obtain an intermediate homogeneous powder,
**b.** a microgranulation step for the intermediate homogeneous powder with the impregnating agent, preferably in solid form, in order to obtain a densified microgranulated powder constituted by uncoated particles corresponding to the abovementioned core,
**c.** optionally a low-temperature drying step of the densified microgranulated powder at a temperature of less than 45°C, in order to obtain a dried densified microgranulated powder,
**d.** optionally a sieving step in order to obtain a sieved densified microgranulated powder,
**e.** a step of coating the uncoated particles of the densified microgranulated powder, optionally dried and optionally sieved, with the sticking agent, preferably in liquid form, in order to obtain a coated powder,
**f.** a drying step of the coated powder in order to obtain a stable powder composition.

16. Pharmaceutical composition containing a stable powder composition containing particles comprising
- a core containing, as active substance, at least one sensitive substance undergoing less than 20% degradation when said powder composition is subjected to temperatures of 60°C to 100°C and/or pressures of 2x10⁶ to 10⁷ Pa and/or a relative humidity of 60% to 100% during the granulation step of said composition, and at least one viscous compound the viscosity of which is greater than 5 Pa.s⁻¹ as impregnating agent for the sensitive substance, and at least a support of the sensitive substance, and
- a coating for the core, which contains at least one viscous compound the viscosity of which is greater than 5 Pa.s⁻¹ as sticking agent,
in which the sensitive substance has at least 80%, in particular at least 90%, of its initial activity, and
said viscous compounds being chosen from carboxymethyl cellulose (CMC), and the viscosity being measured according to the method defined in claim 1,
in combination with a pharmaceutically acceptable vehicle.

17. Food composition comprising a stable powder composition according to anyone of claims 4 to 7, in which the sensitive substance has at least 80%, in particular at least 90%, of its initial activity and is not an enzyme intended for non-human animal food, and at least one food product.

18. Food composition according to claim 17, **characterized in that** it is presented in the form of pellets or granules.
